# EUROPEAN PATENT APPLICATION

(11) **EP 4 154 884 A1**
(43) Date of publication of application: **29.03.2023**
(21) Application number: 21808187.5
(22) Date of filing: 17.05.2021
(51) Int. Cl.: A61K 31/473, A61K 31/4741, A61P 31/14, C07D 221/12, C07D 491/056

(54) **ANTI-SARS-COV-2 DURG**

(30) Priority: 18.05.2020 JP 2020086517
(71) Applicant: Oncolys BioPharma, Inc., Tokyo 105-0001 (JP)
(72) Inventor: BABA Masanori, Kagoshima-shi, Kagoshima 890-8580 (JP); OKAMOTO Mika, Kagoshima-shi, Kagoshima 890-8580 (JP); TOYAMA Masaaki, Kagoshima-shi, Kagoshima 890-8580 (JP); HASHIMOTO Yuichi, Tokyo 161-0032 (JP); HASHIMOTO Hiromasa, Takatsuki-shi, Osaka 569-0084 (JP)
(74) Representative: J A Kemp LLP
(86) International application number: PCT/JP2021/018617
(87) International publication number: WO 2021/235392

(57) **Abstract**

The present invention relates to an anti-SARS-CoV-2 drug including, as an active ingredient, a compound represented by formula (I): 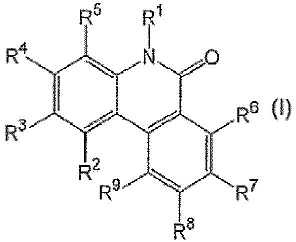 [wherein R¹ is C₁-C₈ alkyl or the like; and R²-R⁹ is hydrogen, halogen, hydroxyl, or Q-(Ci-Cs alkyl), (Q is O, NH, or S) or the like.], or a pharmaceutically acceptable salt thereof.

## Description

### Technical Field

The present invention relates to an anti-SARS-CoV-2 drug.

### Background Art

Coronaviruses are viruses which essentially cause cold symptoms in humans. Four types of coronaviruses have been known, and 10 to 15% of colds are caused by these viruses. In addition, coronaviruses causing Severe Acute Respiratory Syndrome (SARS) and Middle East Respiratory Syndrome (MERS), both having a high fatality rate, have been known so far. The number of patients with SARS is approximately 8,000 with a fatality rate of approximately 10% and the number of patients with MARS is approximately 2,500 with a fatality rate of approximately 35%.

Coronaviruses are positive-stranded RNA viruses having an envelope of approximately 100 nm in diameter. SARS-CoV is classified as a Class II pathogen and MERS-CoV is classified as a Class III pathogen.

SARS-CoV-2 is currently referred to as a new coronavirus, and the infectious disease attributable to the virus has been named COVID-19 by the WHO. It is urgently needed to develop vaccines and therapeutic agents for COVID-19, but it takes some time. Therefore, attempts have been made to repurpose several existing therapeutic agents which were developed with other objectives for the treatment of COVID-19. In the past, the Special Approval for Emergency was granted on May 7, 2020 to remdesivir whose clinical trials had been conducted for the treatment of Ebola virus disease. Other agents such as favipiravir, an anti-influenza agent, have been investigated as a treatment for COVID-19 in clinical trials. These therapeutic agents, however, have not been "optimized" for SARS-CoV-2, which requires solving such problems as insufficient efficacy with normal dosage and adverse effects. Therefore, it is critical to identify and develop a new therapeutic agent which possesses selective and potent antiviral effects against SARS-CoV-2.

Meanwhile, the present inventors have found that phenanthridinone derivatives are effective against human hepatitis C virus and filed a patent application (Patent Literature 1).

However, the relationship between phenanthridinone derivatives and their anti-SARS-CoV-2 activity has not been reported so far.

### Citation List

### Patent Literature

[Patent Literature 1] WO 2011/093483

### Summary of Invention

### Technical Problem

An object of the present invention is to provide an antiviral agent which is effective against SARS-CoV-2.

### Solution to Problem

To attain the above object, the present inventors have established an anti-SARS-CoV-2 assay system for agents and proceeded with screening of various agents. As a result, they have found selective anti-SARS-CoV-2 effects in specific phenanthridinone derivatives, thus completing the present invention.

Specifically, the present invention is summarized as follows.
(1) An anti-SARS-CoV-2 drug comprising, as an active ingredient, a compound represented by formula (I) or a pharmaceutically acceptable salt thereof 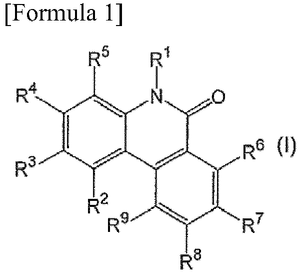 wherein,
   R¹ is selected from among C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(Ci-Cs alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)); and
   R² to R⁹ are each independently selected from Substituent group A consisting of hydrogen, halogen, hydroxyl, NH₂, NO₂, OSO₃H, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), NH-C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), NH-C(NH)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or heterocyclyl), Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl) and Q-(heteroarylalkyl) (wherein Q is O, NH, or S), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or NH₂), NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or heterocyclyl), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-Cs alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)),
   provided that any two of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)), or
   any two of R⁶ to R⁸ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, hydroxyl, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-Cs alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-Cs alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)).
(2) The anti-SARS-CoV-2 drug according to (1), wherein R¹ is C₃-C₇ alkyl.
(3) The anti-SARS-CoV-2 drug according to (1), wherein R¹ is C₄ alkyl.
(4) The anti-SARS-CoV-2 drug according to any one of (1) to (3), wherein R³ is 1,1,1,3,3,3-hexafluoro-2-hydroxypropan-2-yl.
(5) The anti-SARS-CoV-2 drug according to any one of (1) to (4), wherein two groups of R⁴ and R⁵ and/or two groups of R⁷ and R⁸ are methylenedioxy.
(6) The anti-SARS-CoV-2 drug according to any one of (1) to (5), wherein at least one of R² and R⁴ to R⁹ is a group selected from methoxyl, hydroxyl, and halogen.
(7) The anti-SARS-CoV-2 drug according to any one of (1) to (6), which is used for preventing and/or treating COVID-19.

### Advantageous Effects of Invention

The present invention can provide an antiviral agent which is effective against SARS-CoV-2.

### Brief Description of Drawings

[Figure 1] Figure 1 shows the outline of anti-SARS-CoV-2 assay that was implemented in Examples.
[Figure 2] Figure 2 shows the cell changes (cell deaths) induced by SARS-CoV-2 infection and the effect of a phenanthridinone derivative (HA-719) on them.
[Figure 3] Figure 3 shows the relationship between the concentration of phenanthridinone derivatives (HA-719, KZ32, YN029) and the number of viable cells after 3 days of culture in virus-uninfected cells and virus-infected cells.
[Figure 4] Figure 4 shows a ¹H-NMR spectrum of NR-04.
[Figure 5] Figure 5 shows a ¹H-NMR spectrum of NR-32.
[Figure 6] Figure 6 shows a ¹H-NMR spectrum ofNR-51.
[Figure 7] Figure 7 shows a ¹H-NMR spectrum of NR-19.

### Description of Embodiments

Hereafter, preferable embodiments of the present invention are described in detail.

The term "alkyl" used herein refers to a linear or branched aliphatic hydrocarbon group having a specific number of carbon atoms. For example, the term "Ci-Cs alkyl" refers to a linear or branched hydrocarbon chain having at least 1 and up to 8 carbon atoms. Examples of alkyl that can be preferably used include, but are not limited to, methyl, ethyl, n-propyl, isopropyl, n-butyl, sec-butyl, isobutyl, tert-butyl, n-pentyl, n-hexyl, n-heptyl, and n-octyl.

The term "alkenyl" used herein refers to a group resulting from substitution of one or more C-C single bonds of the alkyl with double bonds. Examples of preferable alkenyl include, but are not limited to, vinyl, 1-propenyl, allyl, 1-methylethenyl(isopropenyl), 1-butenyl, 2-butenyl, 3-butenyl, 1-methyl-2-propenyl, 2-methyl-2-propenyl, 1-methyl-1-propenyl, 2-methyl-1-propenyl, 1-pentenyl, 1-hexenyl, n-heptenyl, and 1-octenyl.

The term "alkynyl" used herein refers to a group resulting from substitution of one or more C-C single bonds of the alkyl with triple bonds. Examples of preferable alkynyl include, but are not limited to, ethynyl, 1-propinyl, 2-propinyl, 1-butynyl, 2-butynyl, 3-butynyl, 1-methyl-2-propinyl, 1-pentynyl, 1-hexynyl, 1-heptynyl, and 1-octynyl.

The term "cycloalkyl" used herein refers to alicyclic alkyl having a specific number of carbon atoms. For example, the term "C₃-C₆ cycloalkyl" refers to a cyclic hydrocarbon group having at least 3 and up to 6 carbon atoms. Examples of preferable cycloalkyl include, but are not limited to, cyclopropyl, cyclobutyl, cyclopentyl, and cyclohexyl.

The term "cycloalkenyl" used herein refers to a group resulting from substitution of one or more C-C single bonds of the cycloalkyl with double bonds. Examples of preferable cycloalkenyl include, but are not limited to, cyclopropenyl, cyclobutenyl, cyclopentenyl, and cyclohexenyl.

The term "cycloalkynyl" used herein refers to a group resulting from substitution of one or more C-C single bonds of the cycloalkyl with triple bonds. Examples of preferable cycloalkynyl include, but are not limited to, cyclobutynyl, cyclopentynyl, and cyclohexynyl.

The term "heterocyclyl" used herein refers to a group resulting from substitution of one or more carbon atoms of the cycloalkyl, cycloalkenyl, or cycloalkynyl with a hetero atom or hetero atoms selected from among nitrogen (N), sulfur (S), and oxygen (O). In this case, substitution with N or S includes substitution with N- or S-oxide or dioxide. Examples of preferable heterocyclyl include, but are not limited to, pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, and piperazinyl.

The term "aryl" used herein refers to an aromatic ring group having 6 to 15 carbon atoms. Examples of preferable aryl include, but are not limited to, phenyl, naphthyl, and anthryl (anthracenyl).

The term "arylalkyl" used herein refers to a group resulting from substitution of a hydrogen atom of the alkyl with the aryl. Examples of preferable arylalkyl include, but are not limited to, benzyl, 1-phenethyl, and 2-phenethyl.

The term "arylalkenyl" used herein refers to a group resulting from substitution of a hydrogen atom of the alkenyl with the aryl. An example of preferable arylalkenyl is, but is not limited to, styryl.

The term "heteroaryl" used herein refers to a group resulting from substitution of one or more carbon atoms of the aryl with a hetero atom or hetero atoms selected from among nitrogen (N), sulfur (S), and oxygen (O). In this case, substitution with N or S includes substitution with N- or S-oxide or dioxide. Examples of preferable heteroaryl include, but are not limited to, furanyl, thienyl, pyrrolyl, imidazolyl, pyrazolyl, triazolyl, tetrazolyl, thiazolyl, oxazolyl, isooxazolyl, oxadiazolyl, thiadiazolyl, isothiazolyl, pyridyl, pyridazinyl, pyrazinyl, pyrimidinyl, quinolinyl, isoquinolinyl, and indolyl.

The term "heteroarylalkyl" used herein refers to a group resulting from substitution of a hydrogen atom of the alkyl with the heteroaryl.

The groups described above can be each independently unsubstituted or substituted with one or more groups selected from among halogen, hydroxyl, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S).

The term "halogen" or "halo" used herein refers to fluorine, chlorine, bromine, or iodine.

The term "salt" used herein preferably refers to a pharmaceutically acceptable salt. In this case, examples of preferable counter ions of a compound represented by formula (I) include, but are not limited to, cations such as sodium ions, potassium ions, calcium ions or magnesium ions, or anions such as chloride ions, bromide ions, formate ions, acetate ions, maleate ions, fumarate ions, benzoate ions, ascorbate ions, pamoate ions, succinate ions, bis-methylenesalicylate ions, methanesulfonate ions, ethane disulfonate ions, propionate ions, tartrate ions, salicylate ions, citrate ions, gluconate ions, aspartate ions, stearate ions, palmitate ions, itaconate ions, glycolate ions, p-aminobenzoate ions, glutamate ions, benzenesulfonate ions, cyclohexylsulfamate ions, methanesulfonate ions, ethanesulfonate ions, isethionate ions, benzenesulfonate ions, p-toluenesulfonate ions, naphthalenesulfonate ions, phosphate ions, nitrate ions, sulfate ions, carbonate ions, bicarbonate ions, and perchlorate ions.

In the compound represented by formula (I), R¹ is preferably C₃-C₇ alkyl, more preferably C₄ alkyl, and R³ is preferably 1,1,1,3,3,3-hexafluoro-2-hydroxypropan-2-yl.

Also, in the compound represented by formula (I), a compound in which two groups of R⁴ and R⁵ and/or two groups of R⁷ and R⁸ are methylenedioxy, and a compound in which at least one of R² and R⁴ to R⁹ is a group selected from among methoxyl, hydroxyl and halogen are also a preferred compound.

In the compound represented by formula (I), a compound in which at least one of R⁷ and R⁸ is hydroxyl, O-(C₁-C₈ alkyl), or O-(C₁-C₈ alkyl) with the alkyl moiety substituted with hydroxyl (e.g., OCH₂CH₂OH, OCH₂C(CH₃)₂OH, OCH₂CH₂CH₂OH, OCH₂CH₂CH₂CH₂OH, OCH₂CH₂CH₂CH₂CH₂OH, OCH₂CH(OH)CH₂OH) is preferred.

Among the compounds represented by formula (I), most of the compounds are those specifically disclosed in WO 2011/093483 (Patent Literature 1) and Yuko Nishiyama, "Creation of Bioactive Substance Using Phenanthridinone Skeleton as Alternative to Steroid Skeleton" (URL: http://doi.org/10.15083/00073871). Compounds which are not specifically described in these literatures can also be produced in accordance with the methods described in these literatures.

The product obtained may be purified by a customary method, for example, column chromatography using, e.g., silica gel, as a carrier and a recrystallization method using, e.g., methanol, ethanol, chloroform, dimethyl sulfoxide, n-hexane-ethyl acetate or water. Examples of an elution solvent for column chromatography include methanol, ethanol, chloroform, acetone, hexane, dichloromethane, ethyl acetate and mixed solvents of these.

The compound as mentioned above can be used as an anti-SARS-CoV-2 drug in combination with a customary pharmaceutical carrier. The dosage form thereof is not particularly limited and appropriately selected and used depending on needs. Examples of the dosage form include oral agents such as a tablet, a capsule, a granule, a fine granule, a powder, a sustained release preparation, a liquid preparation, a suspension, an emulsion, a syrup and an elixir and parenteral agents such as an injection and a suppository.

An oral agent is produced by using, for example, starch, lactose, sucrose, mannitol, carboxymethylcellulose and inorganic salts in accordance with an ordinary method. In addition to these components, e.g., a binder, a disintegrant, a surfactant, a lubricant, a glidant, a flavoring agent, a colorant and/or a perfume can be appropriately added.

Examples of the binder include starch, dextrin, gum arabic, gelatin, hydroxypropyl starch, methylcellulose, sodium carboxymethylcellulose, hydroxypropylcellulose, crystalline cellulose, ethylcellulose, polyvinyl pyrrolidone and macrogol.

Examples of the disintegrant include starch, hydroxypropyl starch, sodium carboxymethylcellulose, calcium carboxymethylcellulose, carboxymethylcellulose and a low-substituted hydroxypropylcellulose.

Examples of the surfactant include sodium lauryl sulfate, soy lecithin, sucrose fatty acid ester and polysorbate 80.

Examples of the lubricant include talc, wax, hydrogenated vegetable oil, sucrose fatty acid ester, magnesium stearate, calcium stearate, aluminum stearate and polyethylene glycol.

Examples of the glidant include light anhydrous silicic acid, dry aluminum hydroxide gel, synthetic aluminum silicate and magnesium silicate.

An injection is produced in accordance with an ordinary method. As a diluent, generally, distilled water for injection, saline, a glucose solution, olive oil, sesame oil, peanut oil, soybean oil, corn oil, propylene glycol, polyethylene glycol, and/or the like can be used. If necessary, a disinfectant, a preservative, a stabilizer, an isotonic agent, a soothing agent, and/or the like may be added. In view of stability, an injection can be added in, e.g., a vial, frozen and subjected to ordinary lyophilization to remove a water content. From the lyophilized injection, a liquid preparation can be prepared again immediately before use. The content of a compound of formula (I) in the injection may be varied between the 5 and 50 wt%; however, the content is not limited to this.

Examples of other parenteral agents include a suppository for intrarectal administration, which can be produced in accordance with an ordinary method.

The administration schedule of an anti-SARS-CoV-2 drug formulated varies depending on, e.g., the dosage form and the route of administration, and, for example, can be administered once to four times per day in a period from a week to 3 months.

In order to obtain a desired effect, the dose of an oral agent, which varies depending on the age, body weight and severity of a disease of a patient, is usually, for example, 0.1 to 1000 mg and preferably 1 to 500 mg per adult in terms of the weight of a compound of formula (I), and suitably divided into several portions per day and administered.

In order to obtain a desired effect, the dose of a parenteral agent, which varies depending on the age, body weight and severity of a disease of a patient, is usually, for example, 0.1 to 1000 mg and preferably 1 to 500 mg per adult in terms of the weight of a compound of formula (I), and suitably administered by intravenous injection, intravenous drip infusion, subcutaneous injection, or intramuscular injection.

In addition, the compound represented by formula (I) may be used in combination with other agents which are effective against SARS-CoV-2 infection. These agents can be administered separately in the course of a treatment or combined with a compound represented by formula (1) in a single dosage form such as tablets, intravenous solutions and capsules. Examples of these other agents include remdesivir and favipiravir.

This description includes part or all of the content as disclosed in the description and/or drawing of Japanese Patent Application No. 2020-086517, which is a priority document of the present application.

### Examples

Now, the present invention will be more specifically described below by way of Examples; however, the scope of the present invention is not limited to them.

### [Example 1] Anti-SARS-CoV-2 effect (Assay with VeroE6/TMPRSS2 cells)

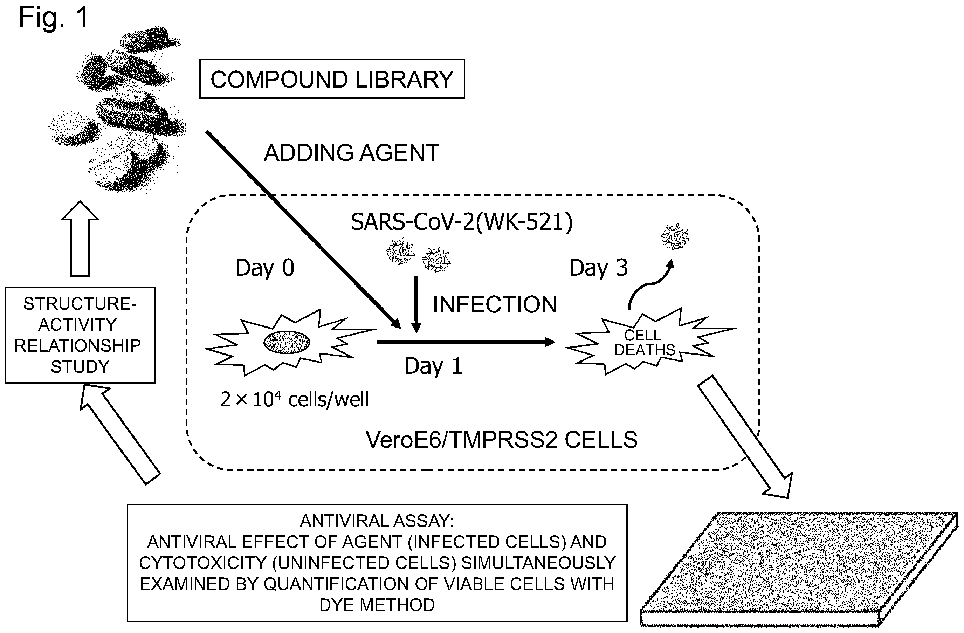

The outline of anti-SARS-CoV-2 assay is shown in Figure 1.

VeroE6/TMPRSS2 cells highly sensitive to SARS-CoV-2 were plated in microplates (2 × 10⁴ cells/well). After 24 hours of culture, agents of various concentrations and SARS-CoV-2 (WK-521) (obtained from the National Institute of Infectious Diseases, Japan) were added with multiplicity of infection (MOI) = 0.01, and then the cells were cultured at 37°C for 3 days. After the culture, 110 µL of culture supernatants were removed, and 10 µL of Cell Counting Kit-8 (DOJINDO LABORATORIES, a viable cell counting kit using water-soluble tetrazolium salt WST-8 (2-(2-methoxy-4-nitrophenyl)-3-(4-nitrophenyl)-5-(2,4-disulfophenyl)-2H-tetrazolium monosodium salt) as a coloring reagent) was added. After 2 hours of culture, 100 µL of hydrochloric acid in 2-propanol was added, mixed adequately and then the absorbance of each well was measured at 450/620 nm. The anti-SARS-CoV-2 effect and cytotoxicity of each agent were determined by comparing the number of viable cells in infected and uninfected cells with those in the absence of the agent.

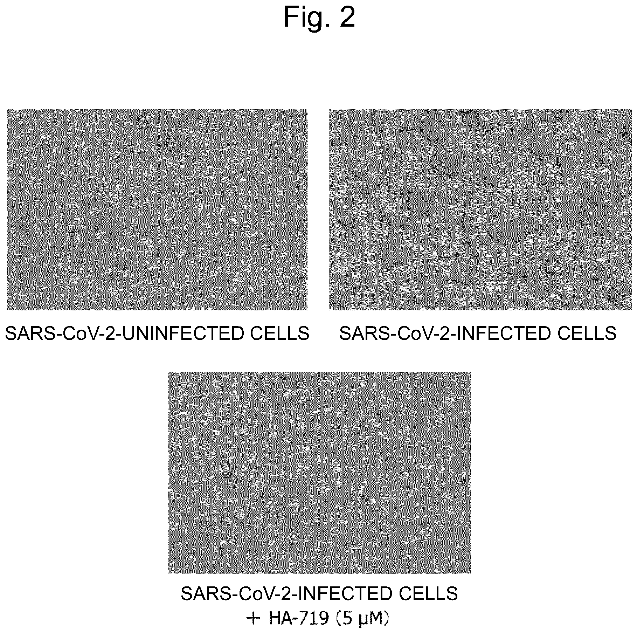

Figure 2 shows the cell changes (cell deaths) induced by SARS-CoV-2 infection and the effect of a phenanthridinone derivative (HA-719) on them observed under a microscope.

Figure 2 reveals that in the absence of the agent almost all the cells were destroyed by SARS-CoV-2 infection (upper-right of Figure 2), while with the presence of 5 µM of HA-719, almost no cell death was observed due to SARS-CoV-2 infection.

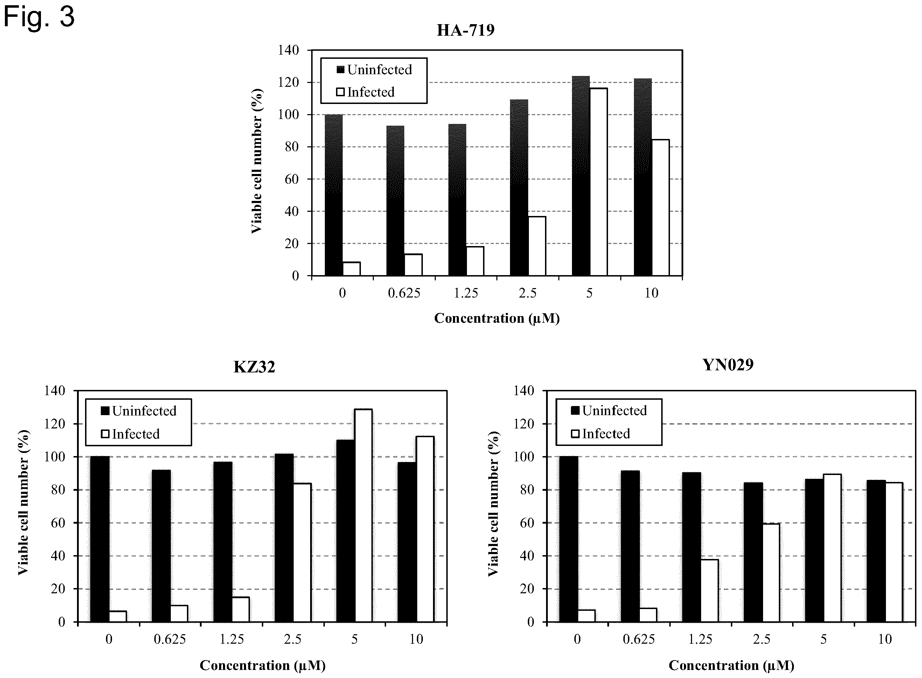

Figure 3 shows the relationship between the concentration of phenanthridinone derivatives (HA-719, KZ32, YN029) and the number of viable cells after 3 days of culture in virus-uninfected cells and virus-infected cells.

In Figure 3, a vertical axis indicates the number of viable cells after 3 days of culture in virus-uninfected cells and virus-infected cells, and a horizontal axis indicates the concentration of agents. The number of viable cells is expressed as 100% in the absence (0) of the agents. As the concentration of agents increases, the number of viable infected cells increases, indicating that these agents have the anti-SARS-CoV-2 effect.

Table 1 shows the anti-SARS-CoV-2 effect of various phenanthridinone derivatives.

**[Table 1]**

| Name | Structure | EC₅₀ (µM) | CC₅₀ (µM) |
|---|---|---|---|
| HA-579 | 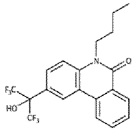 | 8.7 | > 10 |
| HA-718 | 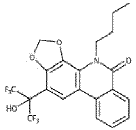 | 3.1 | > 10 |
| HA-719 | 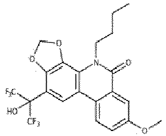 | 2.6 | > 10 |
| HA-727 | 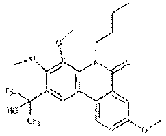 | 4.8 | > 10 |
| HA-754 | 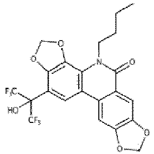 | 5.0 | > 10 |
| KZ01 | 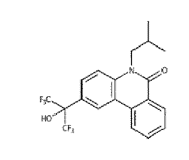 | 5.4 | > 10 |
| KZ07 | 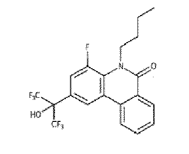 | 3.3 | > 10 |
| KZ08 | 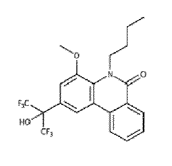 | 2.9 | > 10 |
| KZ12 | 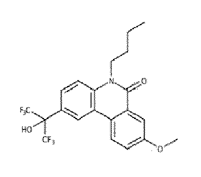 | 8.8 | > 10 |
| KZ15 | 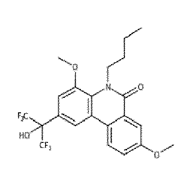 | 3.2 | > 10 |
| KZ24 | 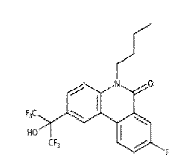 | 4.1 | > 10 |
| KZ26 | 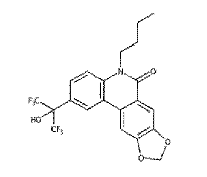 | 2.9 | > 10 |
| KZ31 | 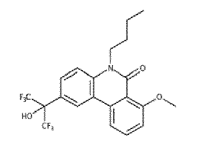 | 3.3 | > 10 |
| KZ32 | 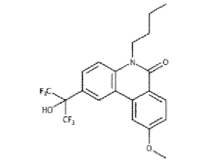 | 1.8 | > 10 |
| KZ33 | 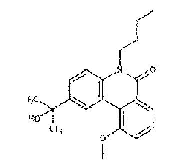 | 4.1 | > 10 |
| KZ39 | 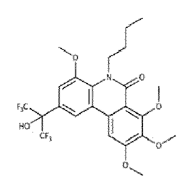 | 8.6 | > 10 |
| YN033 | 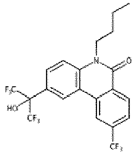 | 6.0 | > 10 |
| YN029 | 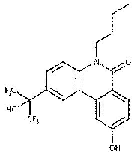 | 1.9 | > 10 |

| | | | |
|---|---|---|---|
| EC₅₀: 50% effective concentration (concentration of agent which inhibits cell death induced by SARS-CoV-2 infection by 50%) CC₅₀: 50% cytotoxic concentration (concentration of agent which reduces the number of viable cells by 50%) | | | |

### [Example 2] Anti-SARS-CoV-2 effect (Assay with HEK293T/ACE2 cells)

### (1) Determination of cell viability (absorbance)

HEK293T/ACE2 cells (2 × 10⁴ cells/well) were plated on microplates. After 24 hours of culture, 50 µL of various agents diluted to 4 times the final concentration was added to each well, and 50 µL of virus solution of SARS-CoV-2 (WK-521, obtained from the National Institute of Infectious Diseases, Japan) was also added with MOI = 0.1 (plate for infection). To a plate for cells, 50 µL of various agents diluted to 4 times the final concentration was added to each well, and 50 µL of cell culture solution was also added. After 3 days of culture, culture supernatants of the plate for infection were transferred to a new plate and stored at - 80°C. From the plate for cells, 110 µL of culture solution was removed and 10 µL of Cell Counting Kit-8 was added. After 2 hours of culture in a CO₂ incubator, absorbance was measured at 450 nm (620 nm).

### (2) qPCR measurement of viral RNA in culture supernatants

To 50 µL of culture supernatants, 50 µL of DNA/RNA Shield (Zymo Research Corporation) was added, and RNA extraction was performed (15 µL) using Quick-RNA Viral 96 Kit (Zymo Research Corporation) according to the specification. The extracted RNA was diluted 10-fold using nuclease-free water, and this was used as an RNA sample. The RNA sample was used to synthesize cDNA using High-Capacity RNA-to-cDNA^{™} Kit (Thermo Fisher Scientific K.K.) (Table 2). The qPCR measurement was performed under the conditions shown in Table 3.

**[Table 2]**

| | Each reaction | | RT reaction | Temp | Time |
|---|---|---|---|---|---|
| 2X RT Buffer | 10.0µL | | Reverse Transcription | 37°C | 60 min |
| 20X RT Enzyme Mix | 1 µL | | RT inactivation | 95°C | 5 min |
| Nuclease-free Water | 4 µL | | Hold | 4°C | indefinite |
| Total volume RT master mix | 15 µL | | | | |
| + RNA sample | 5 µL | | | | |
| Final volume | 20 µL | | | | |

**[Table 3]**

| | Each reaction | | qPCR reaction | Reps | Temp | Time |
|---|---|---|---|---|---|---|
| 2X TaqMan Gene Expression Master Mix | 12.5 µL | | UDG incubation | 1 | 50°C | 2 min |
| Fwd Primer | 0.225 µL | | Enzyme activation | 1 | 95°C | 10 min |
| Rev Primer | 0.225 µL | | PCR (cycle) | 40 | 95°C | 15 sec |
| TaqMan Probe | 0.625 µL | | | | 60°C | 1 min |
| Nuclease-free Water | 6.425 µL | | | | | |
| Total volume qPCR master mix | 20 µL | | | | | |
| + cDNA sample | 5 µL | | | | | |
| Final volume | 25 µL | | | | | |

The results of the evaluation of anti-SARS-CoV-2 effect of various phenanthridinone derivatives are shown in Table 4.

**[Table 4]**

| Name | Structure | IC₅₀(µM) | IC₉₀(µM) | CC₅₀(µM) |
|---|---|---|---|---|
| KZ32 | 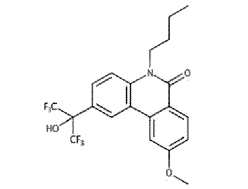 | 1.03 | 7.37 | >20 |
| YN029 | 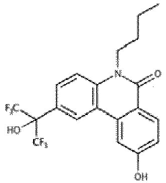 | 0.588 | 3.04 | 16.92 |
| NR-04 | 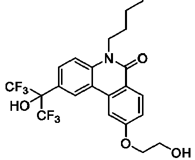 | 0.489 | 3.72 | >20 |
| NR-19 | 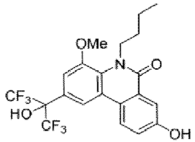 | 0.610 | 4.44 | >20 |
| NR-26 | 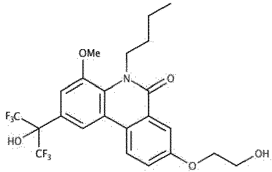 | 1.24 | 14.77 | >20 |
| NR-32 | 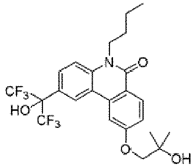 | 0.27 | 2.34 | >20 |
| NR-46 | 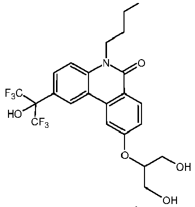 | 0.44 | 3.76 | >20 |
| NR-51 | 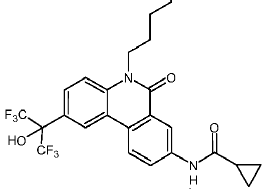 | 0.3 | 1.8 | 8.4 |
| NR-58 | 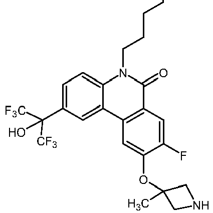 | 1.04 | 4.69 | 10.6 |
| NR-60 | 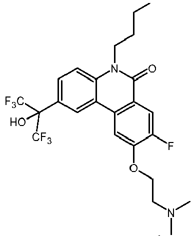 | 0.95 | >10 | 6.73 |
| NR-61 | 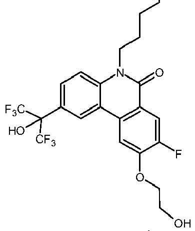 | 1.09 | 4.99 | 19.0 |
| NR-62 | 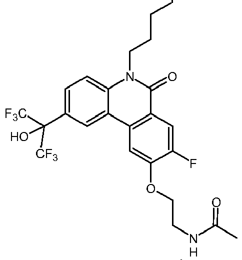 | 1.28 | 7.82 | 13.0 |
| NR-63 | 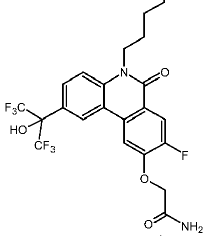 | 0.21 | 2.48 | >20 |
| NR-65 | 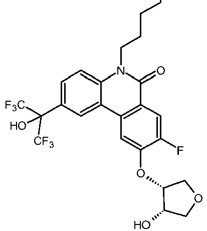 | 0.2 | 6.76 | 18.2 |
| NR-66 | 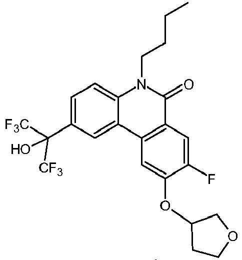 | 0.8 | 5.66 | 13.9 |
| NR-67 | 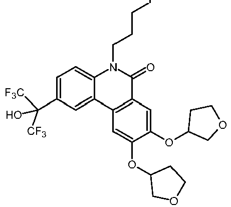 | 1.32 | >10 | 19.0 |
| NR-80 | 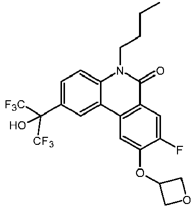 | 0.59 | 6.16 | 19.1 |
| NR-82 | 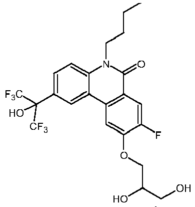 | 0.95 | 8.57 | 14.5 |
| NR-84 | 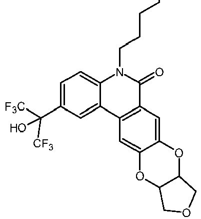 | 1.74 | 5.83 | >20 |
| NR-93 | 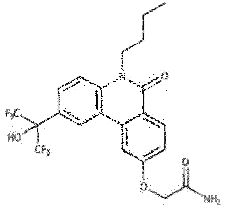 | 0.243 | 3.273 | >20 |
| NR-97 | 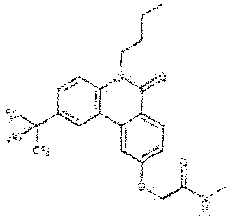 | 0.240 | 3.271 | >20 |

| | | | | |
|---|---|---|---|---|
| IC₅₀: 50% inhibitory concentration (concentration of agent which reduces viral production and replication by 50%) IC₉₀: 90% inhibitory concentration (concentration of agent which reduces viral production and replication by 90%) CC₅₀: 50% cytotoxic concentration (concentration of agent which reduces the number of viable cells by 50%) | | | | |

In Table 4, IC₅₀ and IC₉₀ show the results of qPCR measurement, and CC₅₀ shows the results of the determination of cell viability (absorbance).

Table 1 and Table 4 as well as Figure 2 and Figure 3 show that the phenanthridinone derivatives have the anti-SARS-CoV-2 effect.

The phenanthridinone derivatives were synthesized as described below.

### (Synthesis Example 1)

### Synthesis of 5-butylphenanthridin-6(5H)-one (PN-H)

### (1) Synthesis of 2-iodobenzanilide (a)

Anhydrous dichloromethane (2 ml), triethylamine (105 µl, 753 µmol), and 2-iodobenzoyl chloride (134 mg, 502 µmol) were added to aniline (55.9 mg, 600 µmol), and the mixture was agitated at room temperature for 14 hours. Water was added to the reaction solution, and an organic phase was extracted with ethyl acetate. The organic phase was washed with water, and aqueous solutions of saturated sodium bicarbonate and of saturated sodium chloride, and the resultant was dried over magnesium sulfate, followed by concentration under a reduced pressure. A crude product was purified via silica gel column chromatography using hexane:ethyl acetate (1:1) as an eluting solvent (yield: 160 mg, 99%).

### (2) Synthesis of N-butyl-2-iodobenzanilide (b)

Anhydrous N,N-dimethylformamide (1 ml), 60% sodium hydride (19.5 mg, 488 µmol), and butyl iodide (93 µl, 809 µmol) were added to 2-iodo-N-phenylbenzamide (a) (129 mg, 400 µmol), and the mixture was agitated at room temperature for 21 hours. Water was added to the reaction solution, and an organic phase was extracted with ethyl acetate. The organic phase was washed with water, and aqueous solutions of saturated sodium bicarbonate and of saturated sodium chloride, and the resultant was dried over magnesium sulfate, followed by concentration under a reduced pressure. A crude product was purified via silica gel column chromatography using hexane:ethyl acetate (6:1) as an eluting solvent (yield: 149 mg, 98%).

### (3) Synthesis of 5-butylphenanthridin-6(5H)-one (PN-H)

Anhydrous N,N-dimethylacetamide (1.5 ml), potassium carbonate (82.9 mg, 600 µmol), palladium acetate (3.4 mg, 15.1 µmol), and tri(cyclohexyl)phosphine-tetrafluoroborate (11.3 mg, 30.7 µmol) were added to N-butyl-2-iodo-N-phenylbenzamide (b) (114 mg, 300 µmol), and the mixture was agitated at 130°C for 14 hours. A reaction solution was diluted with ethyl acetate and filtered through celite. The organic phase was washed with water and an aqueous solution of saturated sodium chloride, and the resultant was dried over sodium sulfate, followed by concentration under a reduced pressure. A crude product was purified via silica gel column chromatography using hexane:ethyl acetate (4:1) as an eluting solvent (yield: 75.5 mg, 100%).

FAB-MS m/z 252 (M+H)⁺; ¹H NMR (500 MHz, CDCl₃) δ 8.54 (dd, 1H, J = 7.9, 1.3 Hz), 8.29 (dd, 1H, J = 8.6, 1.3 Hz), 8.27 (d, 1H, J = 8.6 Hz), 7.74 (ddd, 1H, J = 8.0, 7.4, 1.2 Hz), 7.57 (t, 1H, J = 7.4, Hz), 7.53 (ddd, 1H, J = 8.5, 7.4, 1.2 Hz), 7.40 (d, 1H, J = 8.5 Hz), 7.30 (dd, 1H, J = 8.0, 7.4 Hz), 4.38 (t, 2H, J = 7.9, Hz), 1.78 (td, 2H, J = 7.9, 7.3 Hz), 1.52 (sextet, 2H, J = 7.3 Hz), 1.00 (t, 3H, J = 7.3, Hz).

### (Synthesis Example 2)

### Synthesis of 4-butyl-11-(1',1',1',3',3',3'-hexafluoro-2'-hydroxypropan-2'-yl)-7-methoxy-[1,3] dioxolo[4,5-c]phenanthridin-5(4H)-one (HA-719)

The process of synthesis is as shown in scheme below.

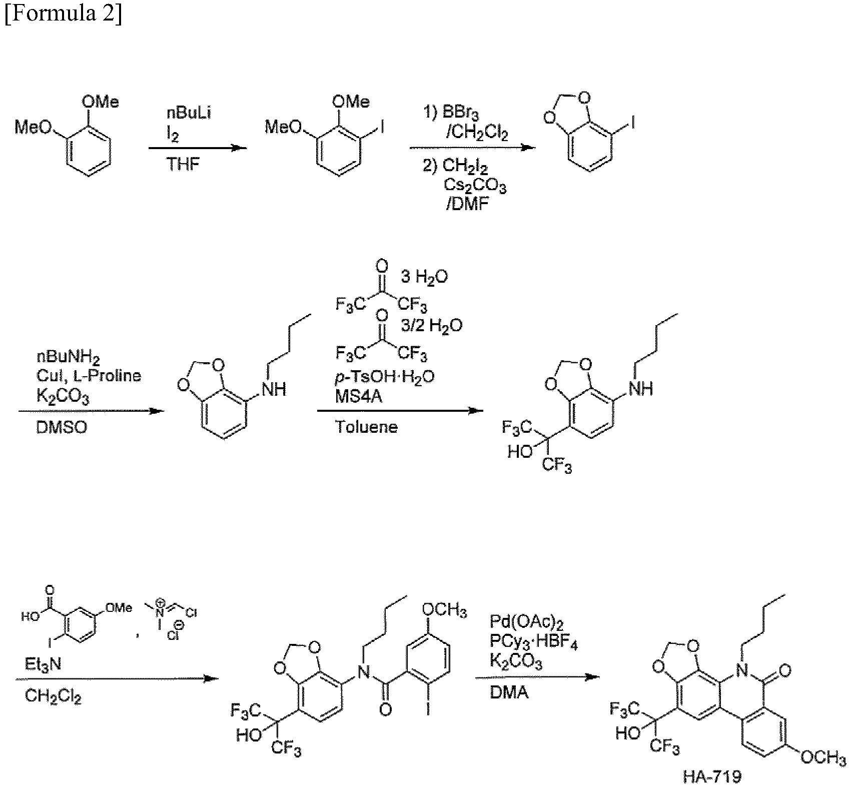

### (1) Synthesis of 3-iodo-1,2-dimethoxybenzene (i)

Anhydrous tetrahydrofuran (10 ml) and n-butyllithium (1.65 M hexane solution, 8.70 ml, 14.4 mmol) were added to 1,2-dimethoxybenzene (1.80 g, 13.0 mmol) at -10°C, and the mixture was agitated at room temperature for 2 hours. The reaction solution was cooled to - 45°C, a solution of iodine (3.63 g, 14.3 mmol) dissolved in 10 ml of anhydrous tetrahydrofuran was added thereto, and the mixture was agitated at room temperature for 1.5 hours. An aqueous solution of saturated ammonium chloride was added to the reaction solution, and an organic phase was extracted with ethyl acetate. The organic phase was washed with an aqueous solution of 10% sodium thiosulfate and an aqueous solution of saturated sodium chloride, and the resultant was dried over sodium sulfate, followed by concentration under a reduced pressure. A crude product was purified via silica gel column chromatography using hexane:ethyl acetate (29:1) as an eluting solvent (yield: 2.44 g, 71%).

### (2) Synthesis of3-iodobenzo[d][1,3]dioxole (j)

Anhydrous dichloromethane (10 ml) and boron tribromide (1.0 M dichloromethane solution, 14.0 ml, 14.0 mmol) were added to 3-iodo-1,2-dimethoxybenzene (i) (792 mg, 3.00 mmol) at -78°C, and the mixture was agitated at room temperature for 18 hours. The reaction solution was poured into iced water, dichloromethane was evaporated under a reduced pressure, and an organic phase was extracted with ethyl acetate. The organic phase was washed with an aqueous solution of saturated sodium chloride, and the resultant was dried over sodium sulfate, followed by concentration under a reduced pressure. Anhydrous N,N-dimethylformamide (30 ml), cesium carbonate (1.00 g, 3.07 mmol), and diiodomethane (0.250 ml, 3.10 mmol) were added to the residue, and the mixture was agitated at 120°C for 1 hour. N,N-dimethylformamide was evaporated under a reduced pressure, water was added thereto, and an organic phase was extracted with ethyl acetate. The organic phase was washed with water and an aqueous solution of saturated sodium chloride, and the resultant was dried over sodium sulfate, followed by concentration under a reduced pressure. A crude product was purified via silica gel column chromatography using hexane:ethyl acetate (50:1) as an eluting solvent (yield: 563 mg, 76%).

### (3) Synthesis of 3-(butylamino)benzo[d][1,3]dioxole (k)

Anhydrous dimethyl sulfoxide (2.0 ml), copper iodide (85.7 mg, 0.450 mmol), L-proline (104 mg, 0.900 mmol), potassium carbonate (415 mg, 3.00 mmol), and butylamine (0.60 ml, 6.07 mmol) were added to 3-iodobenzo[d][1,3]dioxole (j) (372 mg, 1.50 mmol), and the mixture was agitated at 90°C for 11 hours. Ethyl acetate was added to the reaction solution. The resultant was washed with water and an aqueous solution of saturated sodium chloride, dried over sodium sulfate, followed by concentration under a reduced pressure. A crude product was purified via silica gel column chromatography using hexane:ethyl acetate (50:1) as an eluting solvent (yield: 233 mg, 81%).

### (4) Synthesis of 2-[(4-butylamino)benzo[d][1,3]dioxole)]-1,1,1,3,3,3-hexafluoropropan-2-ol (1)

Anhydrous toluene (2.0 ml), hexafluoroacetone sesquihydrate (386 mg, 2.00 mmol), hexafluoroacetone trihydrate (440 mg, 2.00 mmol), p-toluenesulfonic acid monohydrate (19.0 mg, 0.100 mmol), and Molecular Sieve 4A (700 mg) were added to 3-(butylamino)benzo[d][1,3]dioxole (k) (193 mg, 1.00 mmol), and the mixture was agitated at 120°C for 9 hours. Ethyl acetate was added to the reaction solution, and Molecular Sieve 4A was separated by filtration. The organic phase was washed with water and an aqueous solution of saturated sodium chloride, and the resultant was dried over sodium sulfate, followed by concentration under a reduced pressure. A crude product was purified via silica gel column chromatography using hexane:ethyl acetate (19:1) as an eluting solvent (yield: 292 mg, 81%).

### (5) Synthesis of N-butyl-N-[4-(1,1,1,3,3,3-hexafluoro-2-hydroxypropan-2-yl)benzo[d] [1,3] dioxole] -2-iodo-5 -methoxybenzamide (m)

Anhydrous dichloromethane (1.0 ml) and chloromethylenedimethyliminium chloride (76.8 mg, 0.600 mmol) were added to 2-iodo-5-methoxybenzoic acid (167 mg, 0.600 mmol) under ice cooling, and the resultant was agitated at room temperature for 30 minutes. A solution of 2-[(4-butylamino)benzo[d][1,3]dioxole]]-1,1,1,3,3,3-hexafluoropropan-2-ol (1) (72.0 mg, 0.200 mmol) dissolved in triethylamine (0.120 ml, 0.861 mmol) and dichloromethane (0.5 ml) was added to the reaction solution, and the mixture was agitated at room temperature for 20 hours. Water was added to the reaction solution, and an organic phase was extracted with ethyl acetate. The organic phase was washed with an aqueous solution of saturated sodium chloride, and the resultant was dried over sodium sulfate, followed by concentration under a reduced pressure. A crude product was purified via silica gel column chromatography using hexane:ethyl acetate (4: 1) as an eluting solvent (yield: 123 mg, 99%).

### (6) Synthesis of 4-butyl-11-(1',1',1',3',3',3'-hexafluoro-2'-hydroxypropan-2'-yl)-7-methoxy-[1,3]dioxolo[4,5-c]phenanthridin-5(4H)-one (HA-719)

A target compound was obtained in the same manner as in Synthesis Example 1-(3), except for the use of N-butyl-N-[4-(1,1,1,3,3,3-hexafluoro-2-hydroxypropan- 2-yl)benzo[d][1,3]dioxole]-2-iodo-5-methoxybenzamide (m) instead of N-butyl-2-iodo- N-phenylbenzamide (b) (51%).

HRMS (FAB) calculated: C₂₂H₂₀F₆NO₅ 492.1246; measured: 492.1257 (M+H)⁺; ¹H NMR (DMSO-d₆, 500 MHz) δ 8.94 (s, 1H), 8.20 (d, J = 9.2 Hz, 1H), 8.16 (s, 1H), 7.74 (d, J = 3.1 Hz, 1H), 7.46 (dd, J = 9.2, 3.1 Hz, 1H), 6.16 (s, 2H), 4.44 (t, J = 7.9 Hz, 2H), 3.89 (s, 3H), 1.68 (tt, J = 7.3, 7.9 Hz, 2H), 1.36 (qt, J = 7.3, 7.9 Hz, 2H), 0.92 (t, J = 7.9 Hz, 3H).

### (Synthesis Example 3)

### Synthesis of 5-butyl-2-(1',1',1',3',3',3'-hexafluoro-2'-hydroxypropan-2'-yl)-4,8-dimethoxy-phenanthridin-6(5H)-one (KZ15)

A target compound was obtained in the same manner as in Synthesis Example 2, except for the use of N-butyl-2-methoxyaniline instead of 3-(butylamino)benzo[d] [1,3]dioxole (k) and 2-bromo-5-methoxybenzoic acid instead of 2-iodo-5-methoxybenzoic acid.

¹H NMR (500 MHz, CDCl₃): δ 8.18 (s, 1H), 8.14 (d, 1H, J = 9.1 Hz), 7.92 (d, 1H, J = 3.1 Hz), 7.38 (dd, 1H, J = 8.5, 3.1 Hz), 7.23 (brs, 1H), 6.98 (s, 1H), 4.39 (t, 2H, J = 7.3 Hz), 4.11 (s, 3H), 3.96 (s, 3H), 1.82 (quin, 2H, J = 7.3 Hz), 1.55 (sext, 2H, J = 7.3 Hz), 1.05 (t, 3H, J = 7.3 Hz).

### (Synthesis Example 4)

### Synthesis of 5-butyl-2-(1',1',1',3',3',3'-hexafluoro-2'-hydroxypropan-2'-yl)-3,8-dimethoxy-phenanthridin-6(5H)-one (KZ16)

A target compound was obtained in the same manner as in Synthesis Example 2, except for the use of N-butyl-3-methoxyaniline instead of 3-(butylamino)benzo[d] [1,3]dioxole (k) and 2-bromo-5-methoxybenzoic acid instead of 2-iodo-5-methoxybenzoic acid.

¹H NMR (500 MHz, CDCl₃): δ 8.38 (s, 1H), 8.04 (d, 1H, J = 8.5 Hz), 7.93 (d, 1H, J = 3.1 Hz), 7.33 (dd, 1H, J = 8.5, 3.1 Hz), 7.32 (s, 1H), 4.56 (t, 2H, J = 7.3 Hz), 4.34 (s, 1H), 3.97 (s, 3H), 3.95 (s, 3H), 1.85 (quin, 2H, J = 7.3 Hz), 1.45 (sext, 2H, J = 7.3 Hz), 0.99 (t, 3H, J = 7.3 Hz).

### (Synthesis Example 5)

### Synthesis of 5-butyl-2-(1',1',1',3',3',3'-hexafluoro-2'-hydroxypropan-2'-yl)-8,9-dimethoxy-phenanthridin-6(5H)-one (KZ25)

A target compound was obtained in the same manner as in Synthesis Example 2, except for the use of N-butylaniline instead of 3-(butylamino)benzo[d][1,3]dioxole (k) and 2-bromo-4,5-dimethoxybenzoic acid instead of 2-iodo-5-methoxybenzoic acid.

¹H NMR (500 MHz, CDCl₃): δ 8.49 (s, 1H), 7.91 (s, 1H), 7.79 (d, 1H, J = 9.1 Hz), 7.52 (s, 1H), 7.44 (d, 1H, J = 9.1 Hz), 4.41 (brs, 1H), 4.36 (t, 2H, J = 7.3 Hz), 4.07 (s, 3H), 4.03 (s, 3H), 1.77 (quin, 2H, J = 7.3 Hz), 1.51 (sext, 2H, J = 7.3 Hz), 1.01 (t, 3H, J = 7.3 Hz).

### (Synthesis Example 6)

### Synthesis of 5-butyl-2-(1',1',1',3',3',3'-hexafluoro-2'-hydroxypropan-2'-yl)-[1,3] dioxolo[4,5-j] phenanthridin-6(5H)-one (KZ26)

A target compound was obtained in the same manner as in Synthesis Example 2, except for the use of N-butylaniline instead of 3-(butylamino)benzo[d][1,3]dioxole (k) and 2-bromo-4,5-methylenedioxybenzoic acid instead of 2-iodo-5-methoxybenzoic acid.

¹H NMR (500 MHz, CDCl₃): δ 8.43 (s, 1H), 7.84 (s, 1H), 7.79 (d, 1H, J = 9.1 Hz), 7.55 (s, 1H), 7.41 (d, 1H, J = 9.1 Hz), 6.13 (s, 2H), 4.45 (s, 1H), 4.31 (t, 2H, J = 7.3 Hz), 1.75 (quin, 2H, J = 7.3 Hz), 1.50 (sext, 2H, J = 7.3 Hz), 1.01 (t, 3H, J = 7.3 Hz).

### (Synthesis Example 7)

### Synthesis of 5-butyl-2-(1',1',1',3',3',3'-hexafluoro-2'-hydroxypropan-2'-yl)-7,8-dimethoxy-phenanthridin-6(5H)-one (KZ27)

A target compound was obtained in the same manner as in Synthesis Example 2, except for the use of N-butylaniline instead of 3-(butylamino)benzo[d][1,3]dioxole (k) and 2-bromo-5,6-dimethoxybenzoic acid instead of 2-iodo-5-methoxybenzoic acid.

¹H NMR (500 MHz, CDCl₃): δ 8.43 (s, 1H), 7.84 (s, 1H), 7.79 (d, 1H, J = 9.1 Hz), 7.55 (s, 1H), 7.41 (d, 1H, J = 9.1 Hz), 6.13 (s, 2H), 4.45 (s, 1H), 4.31 (t, 2H, J = 7.3 Hz), 1.75 (quin, 2H, J = 7.3 Hz), 1.50 (sext, 2H, J = 7.3 Hz), 1.01 (t, 3H, J = 7.3 Hz).

### (Synthesis Example 8)

### Synthesis of 5-butyl-2-(1',1',1',3',3',3'-hexafluoro-2'-hydroxypropan-2'-yl)-8,9,10-trimethoxy-phenanthridin-6(5H)-one (KZ28)

A target compound was obtained in the same manner as in Synthesis Example 2, except for the use of N-butylaniline instead of 3-(butylamino)benzo[d][1,3]dioxole (k) and 2-bromo-3,4,5-trimethoxybenzoic acid instead of 2-iodo-5-methoxybenzoic acid.

¹H NMR (500 MHz, CDCl₃): δ 9.70 (s, 1H), 7.94(s, 1H), 7.84 (d, 1H, J = 9.1 Hz), 7.46 (d, 1H, J = 9.1 Hz), 4.39 (t, 2H, J = 7.3 Hz), 4.04 (s, 3H), 4.02 (s, 3H), 3.65 (s, 3H), 3.89 (brs, 1H), 1.79 (quin, 2H, J = 7.3 Hz), 1.53 (sext, 2H, J = 7.3 Hz), 1.03 (t, 3H, J = 7.3 Hz).

### (Synthesis Example 9)

### Synthesis of 5-butyl-2-(1',1',1',3',3',3'-hexafluoro-2'-hydroxypropan-2'-yl)-3,4-dimethoxy-phenanthridin-6(5H)-one (HA-726)

The process of synthesis is as shown in scheme below.

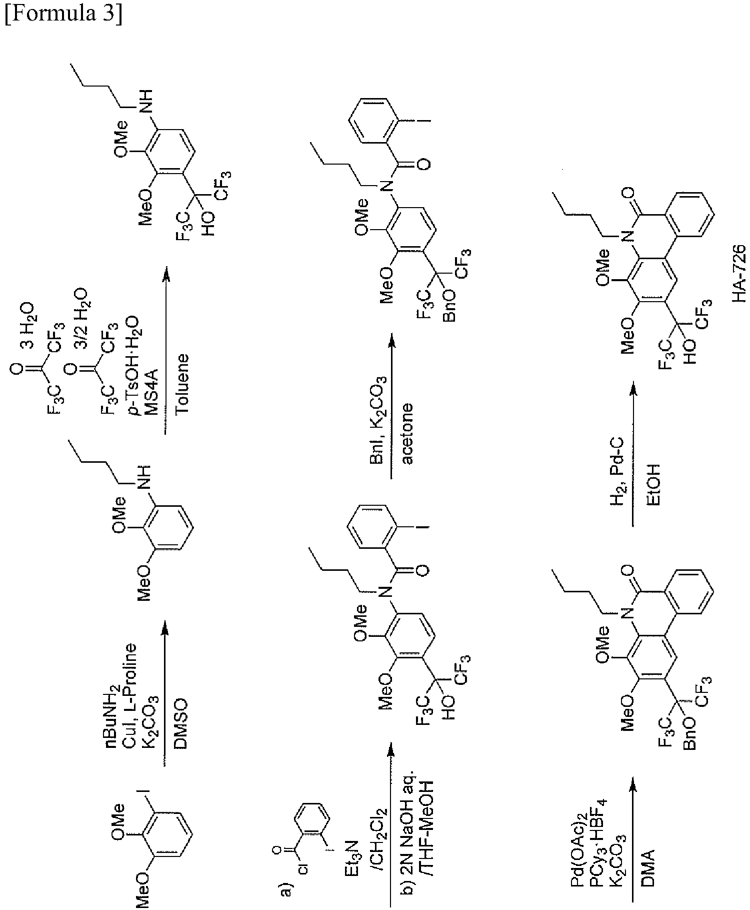

### (1) Synthesis of N-butyl-2,3-dimethoxyaniline (n)

A target compound was obtained in the same manner as in Synthesis Example 2-(3), except for the use of 3-iodo-1,2-dimethoxybenzene (i) instead of 3-iodobenzo[d][1,3]dioxole (j).

### (2) Synthesis of 2-(4-butylamino-2,3-dimethoxyphenyl)-1,1,1,3,3,3-hexafluoropropan-2-ol (o)

A target compound was obtained in the same manner as in Synthesis Example 2-(4), except for the use of N-butyl-2,3-dimethoxyaniline (n) instead of 3-(butylamino)benzo[d][1,3]dioxole (k).

### (3) Synthesis of N-butyl-N-[4-(1,1,1,3,3,3-hexafluoro-2-hydroxypropan-2-yl)-2,3-dimethoxyphenyl]-2-iodobenzamide (p)

Anhydrous dichloromethane (1.0 ml), triethylamine (35.0 µl, 0.251 mmol), and 2-iodobenzoyl chloride (52.1 mg, 0.196 mmol) were added to 2-(4-butylamino-2,3-dimethoxyphenyl)-1,1,1,3,3,3-hexafluoropropan-2-ol (o) (37.2 mg, 0.099 mmol), and the mixture was agitated at room temperature for 12 hours. Dichloromethane was evaporated under a reduced pressure, water was added thereto, and an organic phase was extracted with ethyl acetate. The organic phase was dried over sodium sulfate and then concentrated under a reduced pressure. Tetrahydrofuran (0.3 ml), methanol (0.3 ml), and an aqueous solution of 2 N sodium hydroxide were added to the residue, and the mixture was agitated at room temperature for 2 hours. 2 N hydrochloric acid was added to the reaction solution. The mixture was subjected to extraction with ethyl acetate, drying over sodium sulfate, and then concentration under a reduced pressure. A crude product was purified via silica gel column chromatography using hexane:dichloromethane (1:3) as an eluting solvent (yield: 58.7 mg, 98%).

### (4) Synthesis of N-butyl-N-[4-(2-benzyloxy-1,1,1,3,3,3-hexafluoropropan-2-yl)-2,3-dimethoxyphenyl]-2-iodobenzamide (q)

Acetone (0.8 ml), potassium carbonate (33.2 mg, 0.240 mmol), and benzyl iodide (87.2 mg, 0.400 mmol) were added to N-butyl-N-[4-(1,1,1,3,3,3-hexafluoro- 2-hydroxypropan-2-yl)-2,3-dimethoxyphenyl]-2-iodobenzamide (p) (48.2 mg, 0.080 mmol), and the mixture was agitated at 60°C for 5 hours. Water was added to the reaction solution, and an organic phase was extracted with ethyl acetate. The organic phase was washed with an aqueous solution of saturated sodium chloride, and the resultant was dried over sodium sulfate, followed by concentration under a reduced pressure. A crude product was purified via silica gel column chromatography using hexane:ethyl acetate (6:1) as an eluting solvent (yield: 51.5 mg, 93%).

### (5) Synthesis of 5-butyl-2-(2'-benzyloxy-1',1',1',3',3',3'-hexafluoropropan-2'-yl)-3,4-dimethoxyphenanthridin-6(5H)-one (r)

A target compound was obtained in the same manner as in Synthesis Example 1-(3), except for the use of N-butyl-N-[4-(2-benzyloxy-1,1,1,3,3,3-hexafluoro- propan-2-yl)-2,3-dimethoxyphenyl]-2-iodobenzamide (q) instead of N-butyl-2-iodo-N- phenylbenzamide (c) (77%).

### (6) Synthesis of 5-butyl-2-(1',1',1',3',3',3'-hexafluoro-2'-hydroxypropan-2'-yl)-3,4-dimethoxyphenanthridin-6(5H)-one (HA-726)

Ethanol (0.5 ml) and 7.5% palladium-activated carbon (4.0 mg) were added to 5-butyl-2-(2'-benzyloxy-1',1',1',3',3',3'-hexafluoropropan-2'-yl)-3,4-dimethoxyphenanthridin-6(5H)-one (r) (29.0 mg, 0.051 mmol), and, in a hydrogen atmosphere, the mixture was agitated at room temperature for 6 hours. Dichloromethane was added to the reaction solution. The resultant was filtered through celite, washed with dichloromethane and ethyl acetate, and then concentrated under a reduced pressure. A crude product was purified via silica gel column chromatography using hexane:ethyl acetate (9:1) as an eluting solvent (yield: 24.2 mg, 99%).

HRMS (FAB) calculated: C₂₂H₂₂F₆NO₄ 478.1453; measured: 478.1445 (M+H)⁺; ¹H NMR (DMSO-d₆, 500 MHz) δ 8.76 (s, 1H), 8.50 (s, 1H), 8.33 (d, J = 8.6 Hz, 1H), 8.28 (d, J = 7.3 Hz, 1H), 7.87 (t, J = 8.6 Hz, 1H), 7.64 (t, J = 7.3 Hz, 2H), 4.49 (t, J = 7.3 Hz, 2H), 3.91 (s, 3H), 3.73 (s, 3H), 1.67 (tt, J = 7.3, 7.3 Hz, 2H), 1.28 (qt, J = 7.3, 7.3 Hz, 2H), 0.89 (t, J = 7.3 Hz, 3H).

### (Synthesis Example 10)

### Synthesis of 5-butyl-2-(1',1',1',3',3',3'-hexafluoro-2'-methoxypropan-2'-yl)-1,4-dimethoxy-phenanthridin-6(5H)-one (KZ29)

A target compound was obtained in the same manner as in Synthesis Example 9, except for the use of N-butyl-2,5-dimethoxyaniline instead of N-butyl-2,3-dimethoxyaniline (n) and iodomethane instead of benzyl iodide.

¹H NMR (500 MHz, CDCl₃): δ 8.88 (d, 1H, J = 8.6 Hz), 8.50 (d, 1H, J = 6.7 Hz), 7.72 (dd, 1H, J = 8.6, 6.7 Hz), 7.58 (dd, 1H, J = 8.6, 6.7 Hz), 7.17 (s, 1H), 4.54-4.30 (m, 2H), 3.93 (s, 3H), 3.59 (s, 3H), 3.51 (s, 3H), 1.95-1.80 (m, 2H), 1.45 (sext, 2H, J = 7.3 Hz), 1.00 (t, 3H, J = 7.3 Hz).

### (Synthesis Example 11)

### Synthesis of 5-butyl-2-(1',1',1',3',3',3'-hexafluoro-2'-methoxypropan-2'-yl)-1,4,8-trimethoxyphenanthridin-6(5H)-one (KZ30)

A target compound was obtained in the same manner as in Synthesis Example 10, except for the use of 2-iodo-5-methoxybenzoic acid and chloromethylenedimethyliminium chloride instead of 2-iodobenzoyl chloride and iodomethane instead of benzyl iodide.

¹H NMR (500 MHz, CDCl₃): δ 8.82 (d, 1H, J = 9.2 Hz), 7.93 (d, 1H, J = 3.1 Hz), 7.30 (dd, 1H, J = 9.2, 3.1 Hz), 7.12 (s, 1H), 4.54-4.30 (m, 2H), 3.96 (s, 3H), 3.92 (s, 3H), 3.57 (s, 3H), 3.50 (s, 3H), 1.95-1.80 (m, 2H), 1.46 (sext, 2H, J = 7.3 Hz), 1.01 (t, 3H, J = 7.3 Hz).

### (Synthesis Example 12)

### Synthesis of 5-butyl-2-(1',1',1',3',3',3'-hexafluoro-2'-hydroxypropan-2'-yl)-7-methoxy-phenanthridin-6(5H)-one (KZ31)

A target compound was obtained in the same manner as in Synthesis Example 2, except for the use of N-butylaniline instead of 3-(butylamino)benzo[d][1,3]dioxole (k) and 2-bromo-6-methoxybenzoic acid instead of 2-iodo-5-methoxybenzoic acid.

¹H NMR (500 MHz, CDCl₃): δ 8.64 (s, 1H), 8.37 (d, 1H, J = 9.1 Hz), 7.82 (d, 1H, J = 8.5 Hz), 7.48 (d, 1H, J = 2.4 Hz), 7.37 (d, 1H, J = 8.5 Hz), 7.10 (dd, 1H, J = 9.1, 2.4 Hz), 5.09 (brs, 1H), 4.26 (t, 2H, J = 7.3 Hz), 3.94 (s, 3H), 1.72 (quin, 2H, J = 7.3 Hz), 1.44 (sext, 2H, J = 7.3 Hz), 0.95 (t, 3H, J = 7.3 Hz).

### (Synthesis Example 13)

### Synthesis of 5-butyl-2-(1',1',1',3',3',3'-hexafluoro-2'-hydroxypropan-2'-yl)-9-methoxy-phenanthridin-6(5H)-one (KZ32)

A target compound was obtained in the same manner as in Synthesis Example 2, except for the use of N-butylaniline instead of 3-(butylamino)benzo[d][1,3]dioxole (k) and 2-bromo-4-methoxybenzoic acid instead of 2-iodo-5-methoxybenzoic acid.

¹H NMR (500 MHz, CDCl₃): δ 8.51 (s, 1H), 7.83 (d, 1H, J = 9.1 Hz), 7.81 (d, 1H, J = 7.9 Hz), 7.60 (dd, 1H, J = 7.9, 7.9 Hz), 7.33 (d, 1H, J = 9.1 Hz), 7.06 (d, 1H, J = 7.9 Hz), 5.22 (brs, 1H), 4.20 (t, 2H, J = 7.3 Hz), 4.04 (s, 3H), 1.68 (quin, 2H, J = 7.3 Hz), 1.48 (sext, 2H, J = 7.3 Hz), 0.99 (t, 3H, J = 7.3 Hz).

### (Synthesis Example 14)

### Synthesis of 5-butyl-2-(1',1',1',3',3',3'-hexafluoro-2'-hydroxypropan-2'-yl)-10-methoxy-phenanthridin-6(5H)-one (KZ33)

A target compound was obtained in the same manner as in Synthesis Example 2, except for the use of N-butylaniline instead of 3-(butylamino)benzo[d][1,3]dioxole (k) and 2-bromo-3-methoxybenzoic acid instead of 2-iodo-5-methoxybenzoic acid.

¹H NMR (500 MHz, CDCl₃): δ 9.75 (s, 1H), 8.16 (d, 1H, J = 7.9 Hz), 7.84 (d, 1H, J = 9.2 Hz), 7.50 (dd, 1H, J = 7.9, 7.9 Hz), 7.38 (d, 1H, J = 9.2 Hz), 7.22 (d, 1H, J = 7.9 Hz), 4.68 (brs, 1H), 4.30 (t, 2H, J = 7.3 Hz), 4.01 (s, 3H), 1.74 (quin, 2H, J = 7.3 Hz), 1.49 (sext, 2H, J = 7.3 Hz), 1.00 (t, 3H, J = 7.3 Hz).

### (Synthesis Example 15)

### Synthesis of 5-butyl-2-(1',1',1',3',3',3'-hexafluoro-2'-hydroxypropan-2'-yl)-1-methoxy-phenanthridin-6(5H)-one (KZ34)

A target compound was obtained in the same manner as in Synthesis Example 9, except for the use of N-butyl-3-methoxyaniline instead of N-butyl-2,3-dimethoxyaniline (n).

¹H NMR (500 MHz, CDCl₃): δ 8.88 (d, 1H, J = 8.5 Hz), 8.59 (d, 1H, J = 6.7 Hz), 8.27 (brs, 1H), 7.79 (dd, 1H, J = 8.5, 6.7 Hz), 7.73 (d, 1H, J = 9.8 Hz), 7.65 (dd, 1H, J = 8.5, 6.7 Hz), 7.29 (d, 1H, J = 9.8 Hz), 4.48-4.40 (m, 1H), 4.35-4.4.22 (m, 1H), 3.86 (s, 3H), 1.90-1.73 (m, 2H), 1.60-0.97 (m, 2H), 1.03 (t, 3H, J = 7.3 Hz).

### (Synthesis Example 16)

### Synthesis of 5-butyl-2-(1',1',1',3',3',3'-hexafluoro-2'-hydroxypropan-2'-yl)-1-hydroxy-phenanthridin-6(5H)-one (KZ35) and 5-butyl-2-(1',1',1',3',3',3'-hexafluoro-2'-hydroxypropan-2'-yl)-3-methoxyphenanthridin-6(5H)-one (KZ36)

Target compounds were obtained via separation using column chromatography in the same manner as in Synthesis Example 2, except for the use of N-butyl-3-methoxyaniline instead of 3-(butylamino)benzo[d][1,3]dioxole (k) and 2-iodobenzoyl chloride instead of 2-iodo-5-methoxybenzoic acid.

KZ35: ¹H NMR (500 MHz, CDCl₃): δ 9.52 (d, 1H, J = 8.5 Hz), 8.54 (dd, 1H, J = 7.9, 1.2 Hz), 7.71 (ddd, 1H, J = 8.5, 8.5, 1.2 Hz), 7.53 (dd, 1H, J = 8.5, 7.9 Hz), 7.65 (d, 1H, J = 9.2 Hz), 6.91 (d, 1H, J = 9.2 Hz), 4.36 (t, 2H, J = 7.3 Hz), 3.50-3.20 (m, 2H), 1.80 (quin, 2H, J = 7.3 Hz), 1.52 (sext, 2H, J = 7.3 Hz), 1.03 (t, 3H, J = 7.3 Hz).

KZ36: ¹H NMR (500 MHz, CDCl₃): δ 9.52 (d, 1H, J = 8.5 Hz), 8.54 (dd, 1H, J = 7.9, 1.2 Hz), 7.71 (ddd, 1H, J = 8.5, 8.5, 1.2 Hz), 7.53 (dd, 1H, J = 8.5, 7.9 Hz), 7.65 (d, 1H, J = 9.2 Hz), 6.91 (d, 1H, J = 9.2 Hz), 4.36 (t, 2H, J = 7.3 Hz), 3.50-3.20 (m, 2H), 1.80 (quin, 2H, J = 7.3 Hz), 1.52 (sext, 2H, J = 7.3 Hz), 1.03 (t, 3H, J = 7.3 Hz).

### (Synthesis Example 17)

### Synthesis of 2-(2'-benzyloxy-1',1',1',3',3',3'-hexafluoropropan-2'-yl)-5-butyl-3-methoxy-phenanthridin-6(5H)-one (KZ37)

A target compound was obtained in the same manner as in Synthesis Example 9, except for the use of N-butyl-3-methoxyaniline instead of N-butyl-2,3-dimethoxyaniline (n).

¹H NMR (500 MHz, CDCl₃): δ 8.46 (d, 1H, J = 7.3 Hz), 8.38 (s, 1H), 7.55-7.37 (m, 8H), 6.92 (s, 1H), 4.73 (s, 2H), 4.38 (t, 2H, J = 7.3 Hz), 3.96 (s, 3H), 1.83 (quin, 2H, J = 7.3 Hz), 1.55 (sext, 2H, J = 7.3 Hz), 1.05 (t, 3H, J = 7.3 Hz).

### (Synthesis Example 18)

### Synthesis of 5-butyl-2-(1',1',1',3',3',3'-hexafluoro-2'-hydroxypropan-2'-yl)-7,9-dimethoxy-phenanthridin-6(5H)-one (KZ38)

A target compound was obtained in the same manner as in Synthesis Example 2, except for the use of N-butylaniline instead of 3-(butylamino)benzo[d][1,3]dioxole (k) and 2-bromo-2,4-dimethoxybenzoic acid instead of 2-iodo-5-methoxybenzoic acid.

¹H NMR (500 MHz, CDCl₃): δ 8.47 (s, 1H), 7.78 (d, 1H, J = 8.5 Hz), 7.27 (d, 1H, J = 8.5 Hz), 7.09 (d, 1H, J = 2.4 Hz), 6.59 (d, 1H, J = 2.4 Hz), 5.52 (brs, 1H), 4.20-4.05 (m, 2H), 4.02 (s, 3H), 3.92 (s, 3H), 1.63 (quin, 2H, J = 7.3 Hz), 1.42 (sext, 2H, J = 7.3 Hz), 0.94 (t, 3H, J = 7.3 Hz).

### (Synthesis Example 19)

### Synthesis of 5-butyl-2-(1',1',1',3',3',3'-hexafluoro-2'-hydroxypropan-2'-yl)-7,8,9-trimethoxy-phenanthridin-6(5H)-one (KZ39)

A target compound was obtained in the same manner as in Synthesis Example 2, except for the use of N-butylaniline instead of 3-(butylamino)benzo[d][1,3]dioxole (k) and 2-bromo-4,5,6-trimethoxybenzoic acid instead of 2-iodo-5-methoxybenzoic acid.

¹H NMR (500 MHz, CDCl₃): δ 8.49 (s, 1H), 7.78 (d, 1H, J = 9.2 Hz), 7.46 (s, 1H), 7.39 (d, 1H, J = 9.2 Hz), 4.31 (t, 2H, J = 7.3 Hz), 4.08 (s, 3H), 4.02 (s, 3H), 3.97 (s, 3H), 3.71 (brs, 1H), 1.77 (quin, 2H, J = 7.3 Hz), 1.53 (sext, 2H, J = 7.3 Hz), 1.02 (t, 3H, J = 7.3 Hz).

### (Synthesis Example 20)

### Synthesis of 4-butyl-11-(1',1',1',3',3',3'-hexafluoro-2'-hydroxypropan-2'-yl)-[1,3]dioxolo [4,5-c]phenanthridin-5(4H)-one (HA-718)

A target compound was obtained in the same manner as in Synthesis Example 2, except for the use of 2-iodobenzoyl chloride instead of 2-iodo-5-methoxybenzoic acid and chloromethylenedimethyliminium chloride.

HRMS (FAB) calculated: C₂₁H₁₈F₆NO₄ 462.1140; measured: 462.1169 (M+H)⁺; ¹H NMR (DMSO-d₆, 500 MHz) δ 8.98 (s, 1H), 8.32 (dd, J = 7.9, 1.2 Hz, 1H), 8.27 (s, 1H), 8.26 (d, J = 7.9 Hz, 1H), 7.85 (td, J = 7.3, 1.2 Hz, 1H), 7.62 (t, J = 7.3 Hz, 1H), 6.19 (s, 2H), 4.43 (t, J = 7.9 Hz, 2H), 1.68 (tt, J = 7.3, 7.9 Hz, 2H), 1.37 (qt, J = 7.3, 7.3 Hz, 2H), 0.93 (t, J = 7.3 Hz, 3H).

### (Synthesis Example 21)

### Synthesis of 5-butyl-2-(1',1',1',3',3',3'-hexafluoro-2'-hydroxypropan-2'-yl)-3,4,8-trimethoxy-phenanthridin-6(5H)-one (HA-727)

A target compound was obtained in the same manner as in Synthesis Example 9, except for the use of 2-iodo-5-methoxybenzoic acid and chloromethylenedimethyliminium chloride instead of 2-iodobenzoyl chloride.

HRMS (FAB) calculated: C₂₃H₂₄F₆NO₅ 508.1559; measured: 508.1549 (M+H)⁺; ¹H NMR (DMSO-d₆, 500 MHz) δ 8.72 (s, 1H), 8.40 (s, 1H), 8.21 (d, J = 9.2 Hz, 1H), 7.75 (d, J = 3.1 Hz, 1H), 7.48 (dd, J = 9.2, 3.1 Hz, 1H), 4.50 (t, J = 7.3 Hz, 2H), 3.90 (s, 3H), 3.89 (s, 3H), 3.73 (s, 3H), 1.67 (tt, J = 7.3, 7.3 Hz, 2H), 1.28 (qt, J = 7.3, 7.3 Hz, 2H), 0.89 (t, J = 7.3 Hz, 3H).

### (Synthesis Example 22)

### Synthesis of 11-(2'-benzyloxy-1',1',1',3',3',3'-hexafluoropropan-2'-yl)-4-butyl-7-methoxy-[1,3]dioxolo[4,5-c]phenanthridin-5(4H)-one (HA-758)

A target compound was obtained in the same manner as in Synthesis Example 9-(4), except for the use of 4-butyl-11-(1',1',1',3',3',3'-hexafluoro-2'-hydroxypropan- 2'-yl)-7-methoxy-[1,3]dioxolo[4,5-c]phenanthridin-5(4H)-one (HA-719) instead of N-butyl- N-[4-(2-benzyloxy-1,1,1,3,3,3-hexafluoropropan-2-yl)-2,3-dimethoxyphenyl]-2-iodo- benzamide (q).

HRMS (FAB) calculated: C₂₉H₂₆F₆NO₅ 582.1715; measured: 582.1704 (M+H)⁺; ¹H NMR (DMSO-d₆, 500 MHz) δ 7.80 (s, 1H), 7.73 (d, J = 3.1 Hz, 1H), 7.70 (d, J = 9.2 Hz, 1H), 7.39-7.47 (m, 5H), 7.31 (dd, J = 9.2, 3.1 Hz, 1H), 6.22 (s, 2H), 4.75 (s, 2H), 4.43 (t, J = 7.9 Hz, 2H), 1.69 (tt, J = 7.3, 7.9 Hz, 2H), 1.37 (qt, J = 7.3, 7.3 Hz, 2H), 0.93 (t, J = 7.3 Hz, 3H).

All the compounds shown in Table 1 are known compounds and were produced in accordance with the methods described in the Synthesis Examples above, or in such known literatures as WO 2011/093483 (Patent Literature 1) and Yuko Nishiyama, "Creation of Bioactive Substance Using Phenanthridinone Skeleton as Alternative to Steroid Skeleton" (URL: http://doi.org/10.15083/00073871).

For example, YN029 (5-butyl-2-(1',1',1',3',3',3'-hexafluoro-2'-hydroxypropan-2'-yl)-9-hydroxyphenanthridin-6(5H)-one) is a compound described in Yuko Nishiyama, "Creation of Bioactive Substance Using Phenanthridinone Skeleton as Alternative to Steroid Skeleton" (URL: http://doi.org/10.15083/00073871), can be obtained by treating 5-butyl-2-(1',1',1',3',3',3'-hexafluoro-2'-hydroxypropan-2'-yl)-9-methoxyphenanthridin-6(5H)-one (KZ32) which was obtained in Synthesis Example 13 with boron tribromide, and has the following physical properties.

Mp 250.0-252.5°C

¹H NMR (500 MHz, DMSO-D₆) δ: 8.50 (d, J = 1.7 Hz, 1H), 8.22 (d, J = 9.2 Hz, 1H), 7.81 (d, J= 8.6 Hz, 1H), 7.69 (d, J = 9.2 Hz, 1H), 7.64 (d, J = 2.3 Hz, 1H), 7.10 (dd, J = 8.6, 2.3 Hz, 1H), 4.30 (t, J = 7.7 Hz, 2H), 1.66-1.60 (m, 2H), 1.41 (tq, J = 7.4, 7.4 Hz, 2H), 0.94 (t,J = 7.4 Hz, 3H).

HRMS (FAB) calcd for C₂₀H₁₇F₆NO₃433.1113, found: 433.1118 (M)⁺.

### (Synthesis Example 23)

### Synthesis of 5-butyl-2-(1,1,1,3,3,3-hexafluoro-2-hydroxypropan-2-yl)-9-isopropyloxy-phenanthridin-6(5H)-one (NR-03)

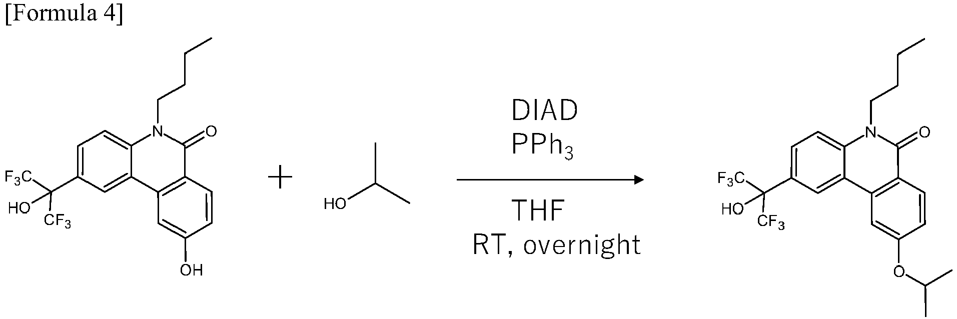

To compound 5d (110 mg) described in Y. Nishiyama, et al., Bioorganic & Medicinal Chemistry 22 (2014) 2799-2808, anhydrous tetrahydrofuran, isopropyl alcohol, diisopropyl azodicarboxylate (DIAD) and triphenylphosphine (PPh₃) were added at 0°C and the mixture was reacted at room temperature overnight to obtain a target compound (yield: 98 mg, 81%).

### (Synthesis Example 24)

### Synthesis of 5-butyl-2-(1,1,1,3,3,3-hexafluoro-2-hydroxypropan-2-yl)-9-(2-hydroxyethoxy)-phenanthridin-6(5H)-one (NR-04)

A process of synthesis is as shown in the following scheme.

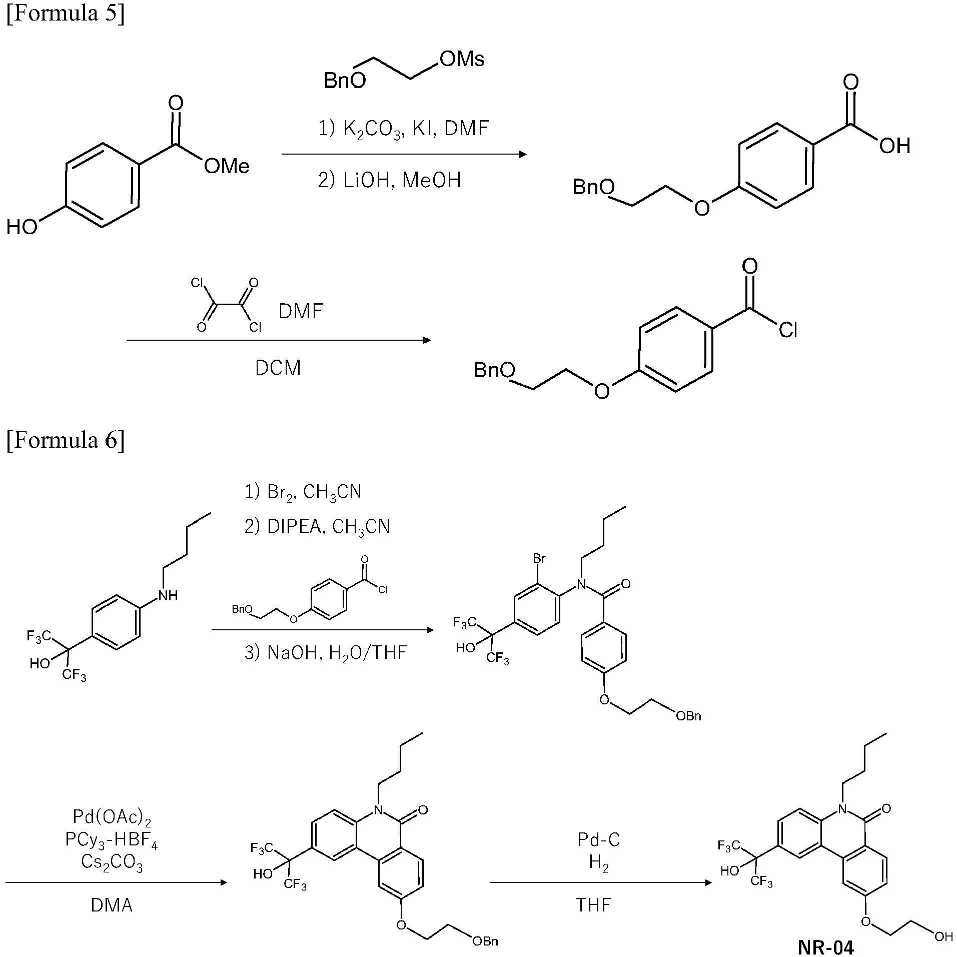

### (1) Synthesis ofNR-04-b

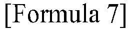

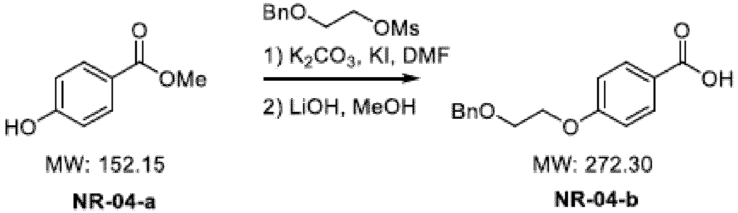

NR-04-a (95.0 g, 624 mmol) was added to N,N-dimethylformamide (1.14 L) under an argon atmosphere. To the reaction solution were added 2-(benzyloxy)ethyl methanesulfonate (169 g, 734 mmol), potassium carbonate (152 g, 1.10 mol) and potassium iodide (24.4 g, 147 mmol) in this order. The reaction solution was agitated at 70°C for 1 hour. N,N-dimethylformamide (100 mL) was added and the mixture was agitated at 85°C for 2 hours. The mixture was cooled to room temperature, MTBE (1.00 L) and city water (2.00 L) were added thereto, and the mixture was subjected to liquid separation. The aqueous phase was extracted with MTBE, and the entire organic phase was washed with a 0.5 N aqueous solution of sodium hydroxide, city water, and an aqueous solution of saturated sodium chloride in this order. The organic phase was dried over sodium sulfate, the drying agent was filtered off, and the filtrate was concentrated to obtain an intermediate.

The entire intermediate was added to methanol (800 mL). An aqueous solution (800 mL) of lithium hydroxide monohydrate (42.0 g, 1.00 mol) was added to the reaction solution, and the mixture was agitated for 2 hours. The solvent was evaporated, city water was added, and the pH was adjusted to 2 using hydrochloric acid. After extracted with ethyl acetate, the entire organic phase was washed with an aqueous solution of saturated sodium chloride. The organic phase was concentrated and the residue was recrystallized from ethyl acetate to obtain NR-04-b (138 g) as a white solid (Y = 81%).

### (2) Synthesis of NR-04-1

### (2-1) Synthesis of an acid chloride of NR-04-b

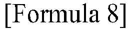

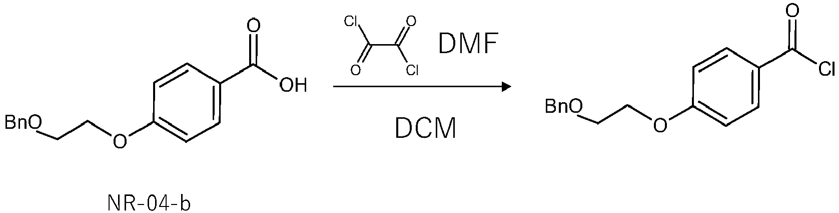

Under an argon atmosphere, NR-04-b (92.9 g, 341 mmol) was added to dichloromethane (527 mL). The reaction solution was immersed in a water bath, and oxalyl chloride (298 mL, 3.41 mol) and N,N-dimethylformamide (0.528 mL, 6.82 mmol) were added thereto. The mixture was agitated at room temperature for 3 hours, and the solvent was evaporated to obtain an acid chloride.

### (2-2) Synthesis of NR-04-1

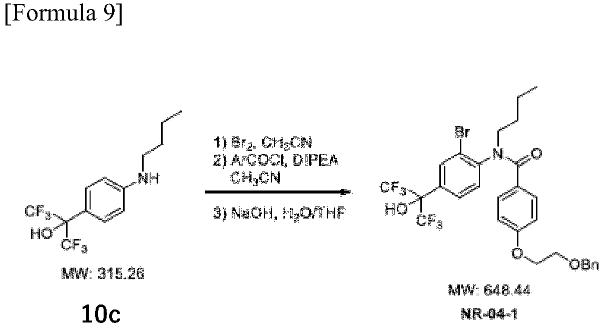

Another flask was prepared and compound 10c (43.0 g, 136 mmol), described in Y. Nishiyama, et al., Bioorganic & Medicinal Chemistry 22 (2014) 2799-2808, was added to acetonitrile (258 mL) under an argon atmosphere. A solution of bromine (23.3 g, 146 mmol)/acetonitrile (38.7 mL) was added dropwise under ice-cooling and the mixture was agitated at room temperature for 4 hours. Under ice-cooling, diisopropylethylamine (119 mL, 682 mmol) was added dropwise, and then a solution of the acid chloride (105 g) prepared above/acetonitrile (258 mL) was added dropwise, and the mixture was agitated for 17 hours. The solvent was evaporated and ethyl acetate was charged instead. The organic phase was washed with an aqueous solution of saturated sodium bicarbonate, and concentrated under a reduced pressure. To the residue were added tetrahydrofuran (861 mL) and an aqueous solution of 2 N sodium hydroxide (205 mL, 205 mmol) and the mixture was agitated for 3 hours. To the reaction solution was added an aqueous solution of 8 N sodium hydroxide (51.3 mL, 205 mmol) and the mixture was agitated for another 6 hours. The solvent was evaporated, and ethyl acetate and city water were added. The pH was adjusted to 4.5 with 2 N hydrochloric acid, and the mixture was subjected to liquid separation. The organic phase was washed with aqueous solutions of saturated sodium bicarbonate and of saturated sodium chloride in this order, and the resultant was dried over sodium sulfate. The drying agent was filtered off and the filtrate was concentrated. IPE was added to the residue and insoluble materials were filtered off. The filtrate was washed with aqueous solutions of saturated sodium bicarbonate and of saturated sodium chloride in this order, and the resultant was dried over sodium sulfate. The residue was purified to obtain NR-04-1 (48.8 g) (Y = 55%).

### (3) Synthesis of NR-04-2

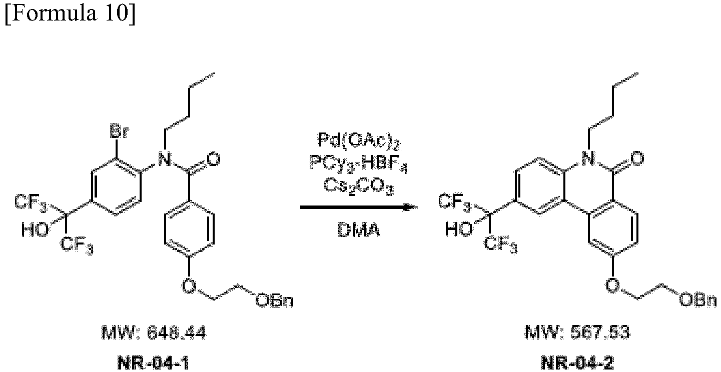

Under an argon atmosphere, NR-04-1 (46.6 g, 67.4 mmol) was added to N,N-dimethylacetamide (438 mL). Cesium carbonate (98.8 g, 303 mmol), palladium(II) acetate (3.03 g, 13.5 mmol) and tricyclohexylphosphine tetrafluoroborate (6.21 g, 16.9 mmol) were added to the reaction solution in this order and the mixture was agitated at 100°C for 3 hours, cooled to room temperature and filtered through celite. The filtrate was poured into city water, the pH was adjusted to 3 to 4 with hydrochloric acid, and then the mixture was subjected to extraction with MTBE. The organic phase was washed with city water and an aqueous solution of saturated sodium chloride in this order, and dried over sodium sulfate. The drying agent was filtered off, and the filtrate was concentrated. The residue was purified to obtain NR-04-2 (25.0 g) (Y = 65%).

### (4) Synthesis of NR-04

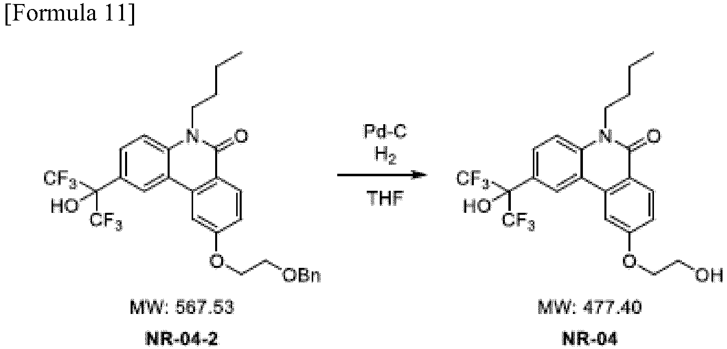

Under an argon atmosphere, NR-04-2 (25.0 g, 44.1 mmol) was added to tetrahydrofuran (375 mL). 5% palladium-carbon (18.8 g, 4.41 mmol, 50% wet product) was added to the reaction solution, and argon within the system was replaced with hydrogen, and then the mixture was agitated at room temperature for 23 hours. Since the raw material was observed to remain, 5% palladium-carbon (10.0 g, 2.35 mmol, 50% wet product) was added to the reaction solution, and the mixture was agitated at room temperature for another 66 hours. The reaction solution was filtered through celite and the filtrate was concentrated. The residue was purified to obtain NR-04 (20.2 g) (Y = 96%).

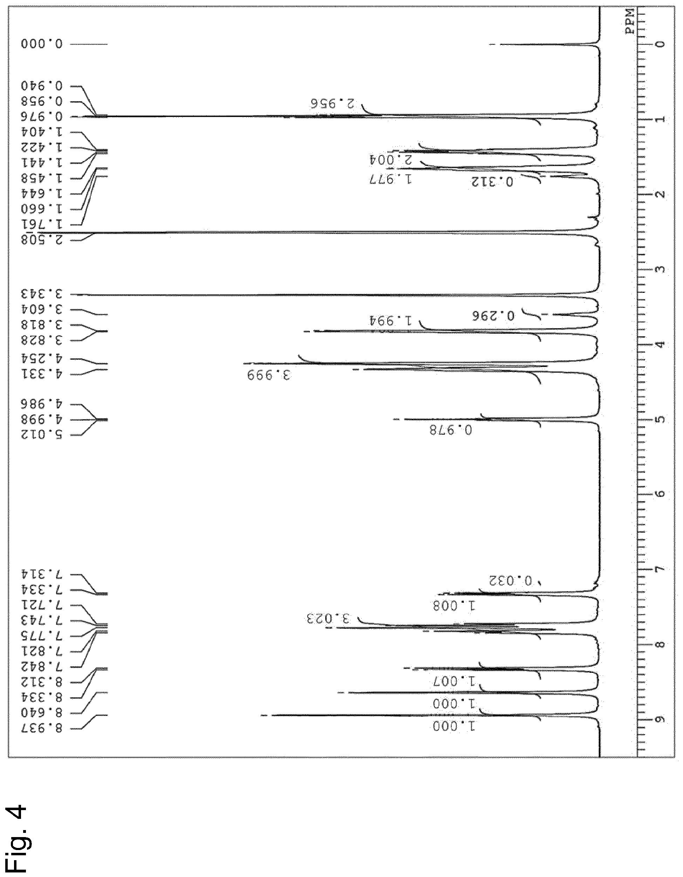

A ¹H-NMR spectrum of NR-04 is shown in Figure 4.

### (Synthesis Example 25)

### Synthesis of 5-butyl-2-(1,1,1,3,3,3-hexafluoro-2-hydroxypropan-2-yl)-9-carboxymethoxy-phenanthridin-6(5H)-one (NR-05)

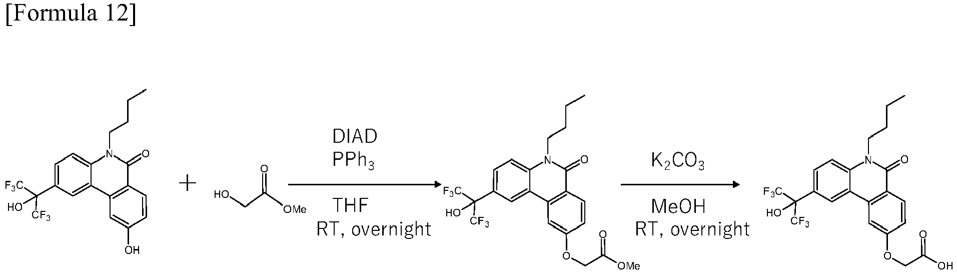

### (1) Synthesis of 5-butyl-2-(1,1,1,3,3,3-hexafluoro-2-hydroxypropan-2-yl)-9-methoxycarbonyl-methoxyphenanthridin-6(5H)-one (NR-08)

A target compound was obtained in the same manner as in Synthesis Example 23, except for the use of methyl glycolate instead of isopropyl alcohol.

### (2) Synthesis of 5-butyl-2-(1,1,1,3,3,3-hexafluoro-2-hydroxypropan-2-yl)-9-carboxymethoxy-phenanthridin-6(5H)-one (NR-05)

Methanol and potassium carbonate were added to 5-butyl-2-(1,1,1,3,3,3-hexafluoro-2-hydroxypropan-2-yl)-9-methoxycarbonylmethoxyphenanthridin-6(5H)-one (NR-08) (60 mg) and allowed to react at room temperature overnight to obtain a target compound (yield: 33 mg, 57%).

### (Synthesis Example 26)

### Synthesis of 5-butyl-2-(1,1,1,3,3,3-hexafluoro-2-hydroxypropan-2-yl)-9-(2-hydroxy-2-methylpropyloxy)phenanthridin-6(5H)-one (NR-32)

A target compound was obtained in the same manner as in Synthesis Example 24, except for the use of 2-benzyloxy-2-methylpropyl alcohol instead of 2-benzyloxyethanol.

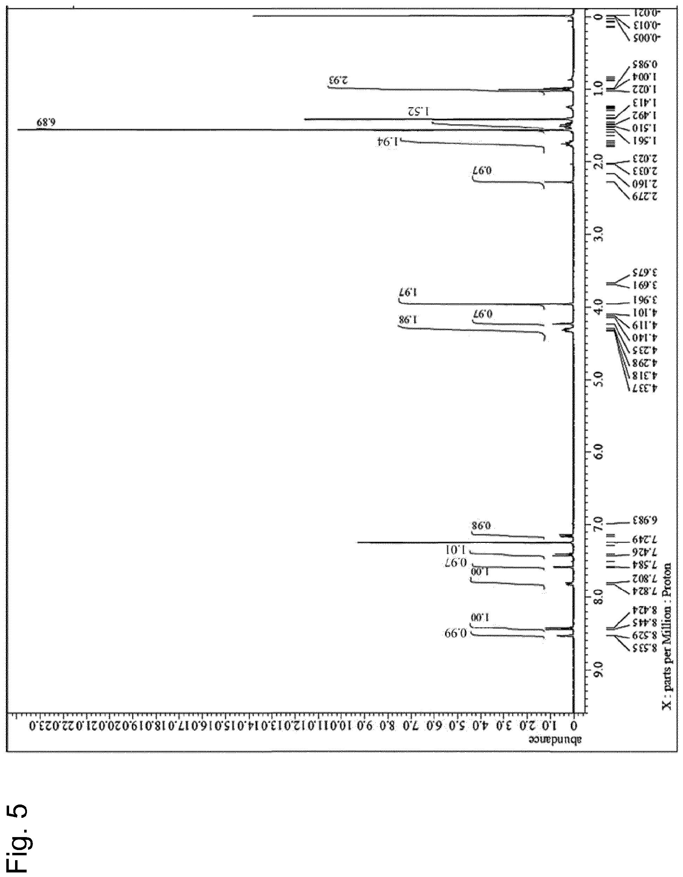

A ¹H-NMR spectrum of NR-32 is shown in Figure 5.

### (Synthesis Example 27)

### Synthesis of NR-51

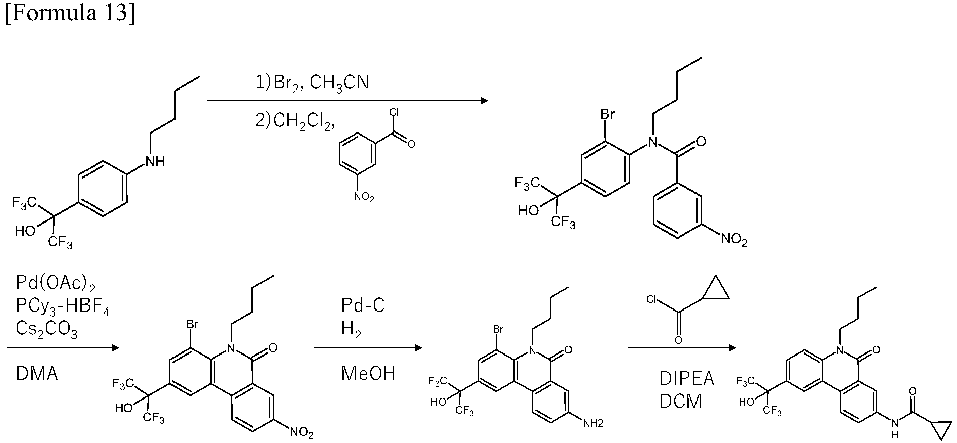

Under an argon atmosphere, compound 10c, described in Y. Nishiyama, et al., Bioorganic & Medicinal Chemistry 22 (2014) 2799-2808, was added to acetonitrile. A solution of bromine/acetonitrile was added dropwise under ice-cooling and the mixture was agitated at room temperature for 1 hour. Dichloromethane, followed by m-nitrobenzoyl chloride, was added in portions and the mixture was agitated at room temperature overnight. Sodium hydroxide and acetonitrile were added, and allowed to react at room temperature overnight to obtain intermediate 1. Under an argon atmosphere, intermediate 1 was added to N,N-dimethylacetamide. Cesium carbonate, palladium(II) acetate and tricyclohexylphosphine tetrafluoroborate were added to the reaction solution in this order, and was agitated at 100°C for 1 hour to obtain intermediate 2. Under an argon atmosphere, intermediate 2 was added to methanol. Palladium-carbon was added to the reaction solution, and argon was replaced with hydrogen, and then the mixture was agitated at room temperature for 1 hour to obtain intermediate 3. To intermediate 3, cyclopropanecarbonyl chloride, dichloromethane and N,N-diisopropylethylamine were added and the mixture was allowed to react in the presence of methanol and potassium carbonate to obtain NR-51.

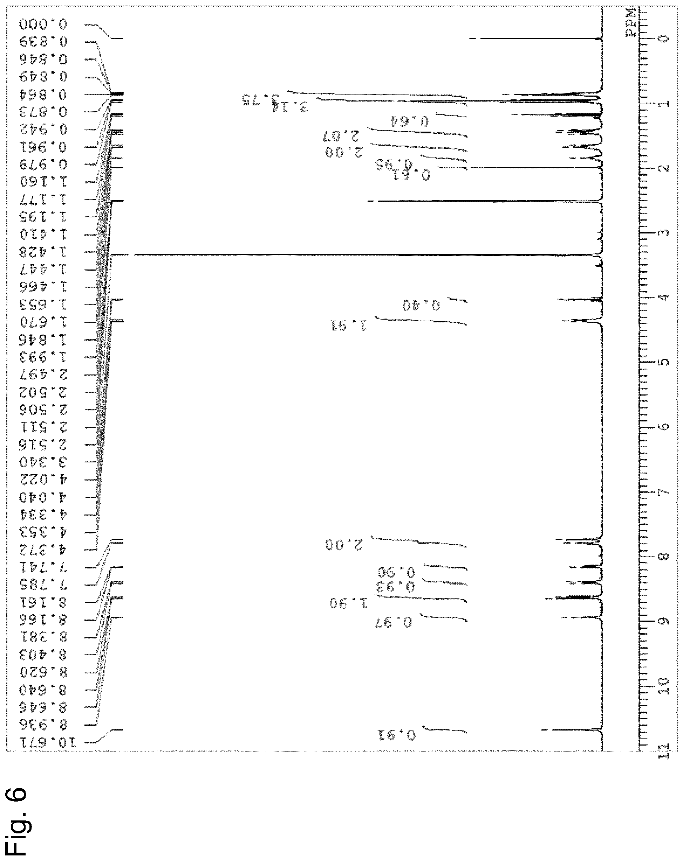

A ¹H-NMR spectrum of NR-51 is shown in Figure 6.

### (Synthesis Example 28)

Other compounds listed in Table 4 were synthesized according to Synthesis Examples 1 to 26 or known methods.

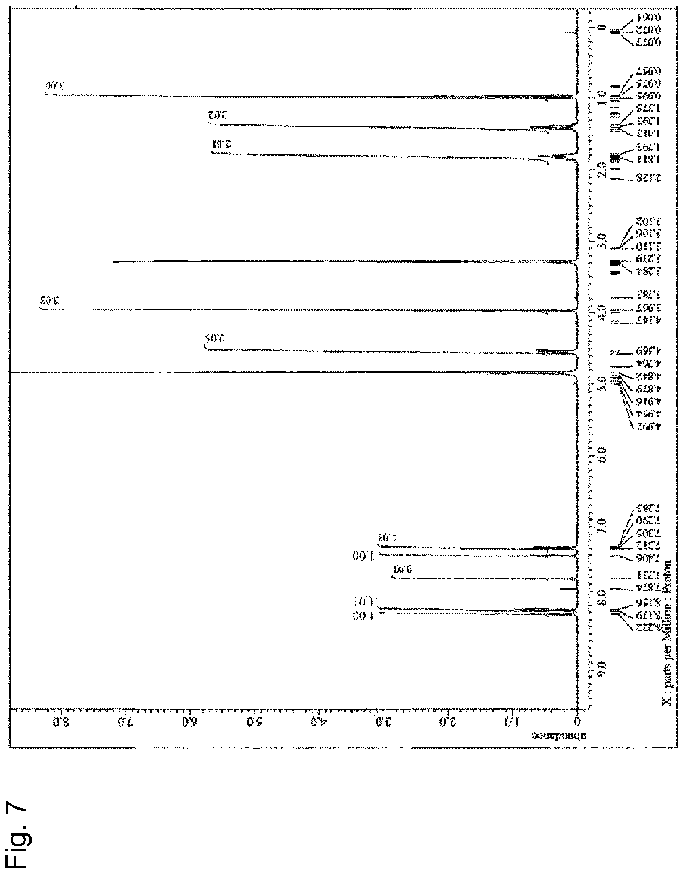

A ¹H-NMR spectrum of NR-19 is shown in Figure 7.

All publications, patents, and patent applications cited herein are incorporated herein by reference in their entirety.

## Claims

1. An anti-SARS-CoV-2 drug comprising, as an active ingredient, a compound represented by formula (I) or a pharmaceutically acceptable salt thereof 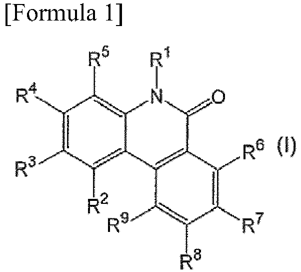 wherein,
R¹ is selected from among Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or NH₂), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(Ci-Cs alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)); and
R² to R⁹ are each independently selected from Substituent group A consisting of hydrogen, halogen, hydroxyl, NH₂, NO₂, OSO₃H, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), NH-C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), NH-C(NH)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), N(R¹⁰)(R¹¹) (wherein R¹⁰ and R¹¹ are each independently Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or heterocyclyl), Q-(Ci-Cs alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl) and Q-(heteroarylalkyl) (wherein Q is O, NH, or S), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or NH₂), NH-C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), N(R¹⁰)(R¹¹) (wherein R¹⁰ and R¹¹ are each independently Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or heterocyclyl), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(Ci-Cs alkyl), Q-(C₂-Cs alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)),
provided that any two of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or NH₂), Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(Ci-Cs alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)), or
any two of R⁶ to R⁸ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, hydroxyl, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), Ci-Cs alkyl, C₂-Cs alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(Ci-Cs alkyl), Q-(C₂-Cs alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)).

2. The anti-SARS-CoV-2 drug according to claim 1, wherein R¹ is C₃-C₇ alkyl.

3. The anti-SARS-CoV-2 drug according to claim 1, wherein R¹ is C₄ alkyl.

4. The anti-SARS-CoV-2 drug according to any one of claims 1 to 3, wherein R³ is 1,1,1,3,3,3-hexafluoro-2-hydroxypropan-2-yl.

5. The anti-SARS-CoV-2 drug according to any one of claims 1 to 4, wherein two groups of R⁴ and R⁵ and/or two groups of R⁷ and R⁸ are methylenedioxy.

6. The anti-SARS-CoV-2 drug according to any one of claims 1 to 5, wherein at least one of R² and R⁴ to R⁹ is a group selected from methoxyl, hydroxyl, and halogen.

7. The anti-SARS-CoV-2 drug according to any one of claims 1 to 6, which is used for preventing and/or treating COVID-19.

8. A compound represented by formula (I) or a pharmaceutically acceptable salt thereof 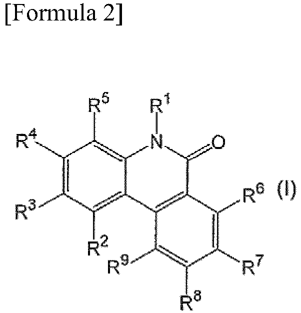 wherein,
R¹ is selected from among C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, hydroxyl, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(Ci-Cs alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)); and
R² to R⁹ are each independently selected from Substituent group A consisting of hydrogen, halogen, hydroxyl, NH₂, NO₂, OSO₃H, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), NH-C(NH)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), N(R¹⁰)(R¹¹) (wherein R¹⁰ and R¹¹ are each independently Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or heterocyclyl), Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl) and Q-(heteroarylalkyl) (wherein Q is O, NH, or S), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, hydroxyl, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), NH-C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, or NH₂), N(R¹⁰)(R¹¹) (wherein R¹⁰ and R¹¹ are each independently C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or heterocyclyl), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-Cs alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)),
provided that any two of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, hydroxyl, NH₂, NO₂, C(O)Z (wherein Z is hydrogen, hydroxyl, Ci-Cs alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O, NH, or S)), and
at least any of R⁷ and R⁸ is hydroxyl, O-(C₁-C₈ alkyl), or O-(C₁-C₈ alkyl) wherein the alkyl moiety is substituted with hydroxyl.
